# EUROPEAN PATENT APPLICATION

(11) **EP 4 316 522 A2**
(43) Date of publication of application: **07.02.2024**
(21) Application number: 23208953.2
(22) Date of filing: 06.11.2018
(51) Int. Cl.: A61K 47/38

(54) **INJECTABLE ISOXAZOLINE PHARMACEUTICAL COMPOSITIONS AND THEIR USE AGAINST PARASITE INFESTATION**

(30) Priority: 07.11.2017 US 201762582381 P; 21.12.2017 US 201762608904 P
(62) Divisional of application: 18799511.3
(71) Applicant: Intervet International B.V., 5831 AN Boxmeer (NL)
(72) Inventor: FREEHAUF, Keith, A, Madison, 07940 (US); CARRILLO, Brian, Rahway, NJ 07065 (US)
(74) Representative: Intervet International B.V.

(57) **Abstract**

An injectable pharmaceutical composition comprising an isoxazoline compound of Formula (I) 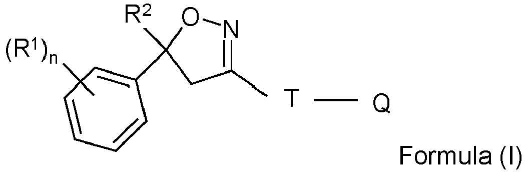 or a salt or N-oxide thereof wherein the isoxazoline compound has a particle size of from about 25 microns to about 250 microns and a method of preventing or treating a parasite infestation using the same.

## Description

### Background

Isoxazoline compounds are known in the art and these compounds and their use as antiparasitic are described, for example, in US patent application US 2007/0066617, and International Patent applications WO 2005/085216, WO 2007/079162, WO 2009/002809, WO 2009/024541, WO 2009/003075, WO 2010/070068 and WO 2010/079077, the disclosures of which, as well as the references cited herein, are incorporated by reference. This class of compounds is known to possess excellent activity against ectoparasites, i.e., parasitic insect and acarids, such as fleas and ticks .

Examples of isoxazoline compounds are carbamoyl benzamide phenyl isoxazoline (CBPI) compounds. A specific example of a CBPI compound is 4-[5-(3,5-Dichlorophenyl)-5-trifluoromethyl-4,5-dihydroisoxazol-3-yl]-2-methyl-N-[(2,2,2-trifluoro-ethylcarbamoyl)-methyl]-benzamide (CAS RN [864731-61-3]) - USAN fluralaner.

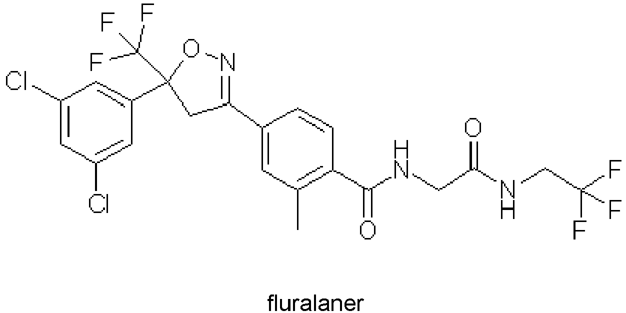

The CBPI compound fluralaner is disclosed in patent application WO 2005/085216.

WO2015/048371 discloses long acting injectable compositions comprising spirocyclic isoxazoline compounds, one biopolymer and at least one carrier, solvent or excipient.

WO2016/138339 discloses long acting injectable formulations for comprising at least one isoxazoline active agent, a poloxamer and a co-solvent. There is no disclosure of formulations that are suspensions or the particle size of the isoxazoline.

WO2016/164487 discloses extended release injectable veterinary formulations comprising at least one isoxazoline active agent, a pharmaceutically acceptable polymer and a solvent for use against parasites. There is no disclosure of formulations that are suspensions or the particle size of the isoxazoline.

U.S. Patent No. 9,609,869 discloses insecticidal compounds based on isoxazoline derivatives for use in controlling pest associated with agriculture, horticulture, animal husbandry and companion animals. There is no disclosure of injectable formulations or administration to animals.

US Patent Application Publication No. 2017/0239218 discloses long acting injectable compositions for combating parasites comprising at least one isoxazoline active agent, a liquid PEG and/or a neutral oil. There is no disclosure of formulations that are suspensions or the particle size of the isoxazoline.

None of these references disclose a solution to the problem of identifying injectable isoxazoline compositions with long term efficacy against parasites and reduced risk of injection site irritation.

### Summary of the Invention

Accordingly, the present invention provides injectable isoxazoline compositions with long term efficacy against parasites and reduced risk of injection site irritation.

An embodiment of the invention is an injectable pharmaceutical composition comprising particles of an isoxazoline compound of Formula (I) 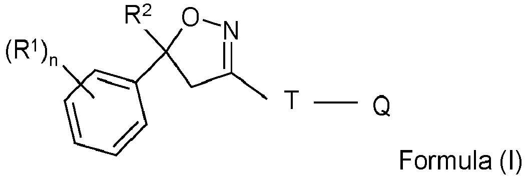 wherein
R¹ is halogen, CF₃, OCF₃, or CN;
n is an integer from 0 up to and including 3, preferably 1, 2 or 3:
m is 1 or 2;
R² is C₁-C₃ haloalkyl, CF₃ or CF₂Cl
T is a 5, or 6 membered ring, or bicyclic, which is optionally substituted by one or more radicals Y;
Y is methyl, halomethyl, halogen, CN, NO₂, NH₂-C=S, or two adjacent radicals Y together form a chain;
Q is X-NR³R⁴, NR⁵-NR⁶-X-R³, X-R³, or a 5-membered N-heteroaryl ring, which is optionally substituted by one or more radicals;
X is CH₂, CH(CH₃), CH(CN), CO, CS;
R³ is hydrogen, methyl, haloethyl, halopropyl, halobutyl, methoxymethyl, methoxyethyl, halomethoxymethyl, ethoxymethyl, haloethoxymethyl, propoxymethyl, ethylaminocarbonylmethyl, ethylaminocarbonylethyl, dimethoxyethyl, propynylaminocarbonylmethyl, N-phenyl-N-methyl-amino, haloethylaminocarbonylmethyl, haloethylaminocarbonylethyl, tetrahydrofuryl, methylaminocarbonylmethyl, (N,N-dimethylamino)-carbonylmethyl, propylaminocarbonylmethyl, cyclopropylaminocarbonylmethyl, propenylaminocarbonylmethyl, haloethylaminocarbonylcyclopropyl, alkylsulfanylalkyl, alkylsulfinylalkyl, alkylsulfonylalkyl, cycloalkyl, 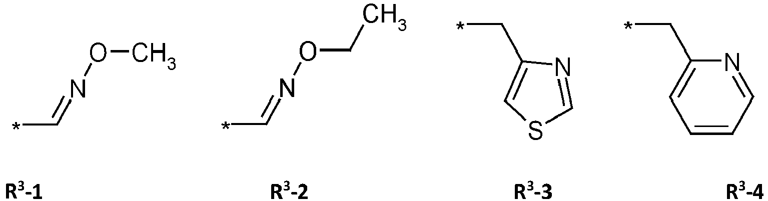 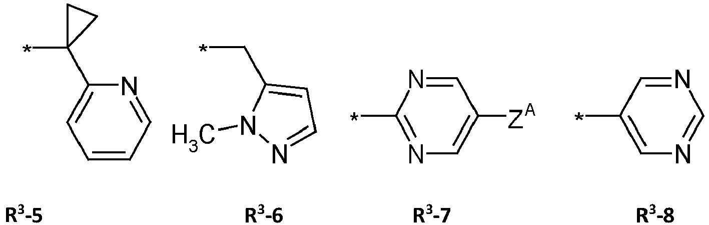 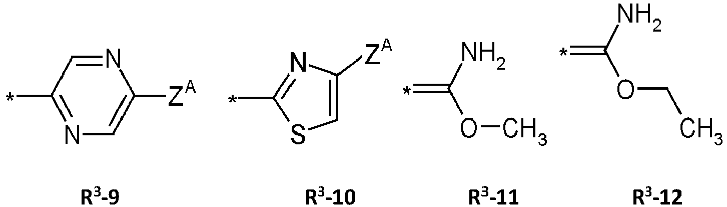 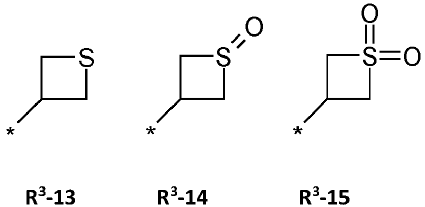 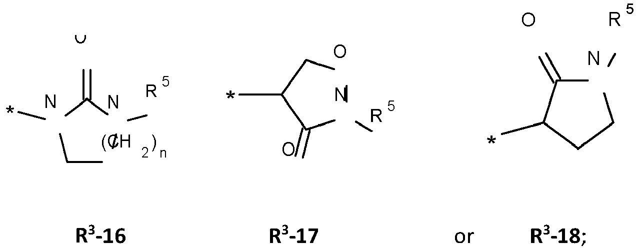
wherein
Z^{A} is hydrogen, halogen, cyano, or halomethyl (CF₃);
R⁴ is hydrogen, ethyl, methoxymethyl, halomethoxymethyl, ethoxymethyl, haloethoxymethyl, propoxymethyl, methylcarbonyl, ethylcarbonyl, propylcarbonyl, cyclopropylcarbonyl, methoxycarbonyl, methoxymethylcarbonyl, aminocarbonyl, ethylaminocarbonylmethyl, ethylaminocarbonylethyl, dimethoxyethyl, propynylaminocarbonylmethyl, haloethylaminocarbonylmethyl, cyanomethylaminocarbonylmethyl, or haloethylaminocarbonylethyl;
R⁵ is H, alkyl, or haloalkyl;
R⁶ is H, alkyl, or haloalkyl;
or wherein R³ and R⁴ together form a substituent selected from the group consisting of: 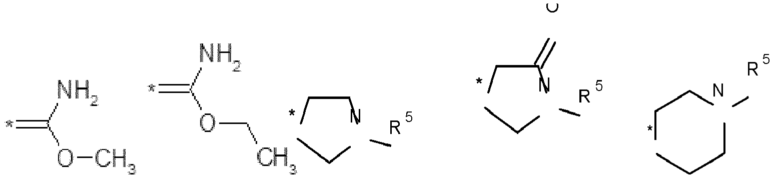 and 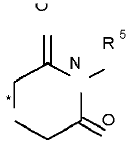
or a salt or N-oxide thereof and a pharmaceutical acceptable excipient wherein the isoxazoline compound has a volume weighted particle size distribution D50 as measured by a static light scattering instrument of from about 25 microns to about 250 microns.

An additional embodiment is a method of treating or preventing a parasite infestation in an animal comprising administering to an animal in need thereof an effective amount of the above injectable pharmaceutical composition.

### Description of the Figures

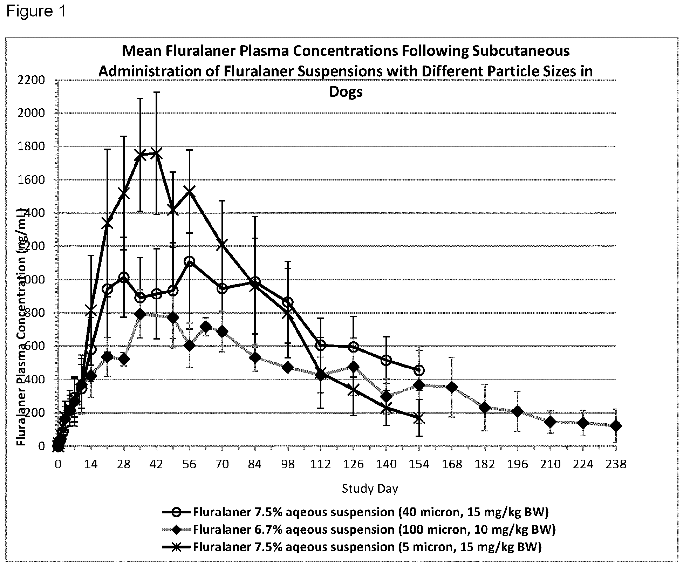

Figure 1 - Mean Fluralaner Plasma Concentrations Following Subcutaneous Administration of Fluralaner Large Particle Suspensions in Dogs
Figure 2 - Mean Fluralaner Plasma Concentrations to demonstrate the effects of irradiation and the absence of poloxamer
Figure 3 - Mean Fluralaner Plasma Concentrations of injectable formulations with different particle size distributions.
Figure 4- Mean Fluralaner Plasma Concentrations to determine the effect of varying concentration of NaCMC and poloxamer as well as different D50 particle sizes.

### Detailed Description

It has been found that the inventive injectable compositions comprising particles of isoxazoline compounds with a defined particle size show desirable bioavailability and duration of efficacy, while causing minimal irritation at the injection site. The compositions also provide desirable safety profiles toward the warm-blooded and bird animal recipients. In addition, it has been discovered that a single administration of such compositions generally provides potent activity against one or more parasites (e.g., ectoparasites, e.g. fleas, ticks or mites), while also tending to provide fast onset of activity, long duration of activity, and/or desirable safety profiles.

The invention also provides methods for the treatment or prevention of parasitic infections and infestations in animals, comprising administering an effective amount of injectable compositions comprising an antiparasitic effective amount of at least one isoxazoline compound of a defined particle size together with a pharmaceutically acceptable excipient. Surprisingly, it has been found that the inventive isoxazoline-containing compositions described herein exhibit superior broad spectrum efficacy against harmful parasites (e.g. ectoparasites such as fleas and ticks) more rapidly, and over a long duration compared to other injectable compositions containing isoxazoline active agents known in the art while exhibiting minimal irritation at the injection site. The pharmaceutical composition of the current invention can be administered by subcutaneous or intramuscular injection. Intravenous injection is possible as well.

### Definitions

Isoxazoline compounds are known in the art and compounds from this class are known to possess excellent activity against parasite infestations, such as ticks and fleas. Embodiments of various isoxazoline compounds of the subject invention are provided below.

Injection site irritation is the injury produced at the injection site and surrounding tissue when an animal receives an injection of a pharmaceutical composition. Such injury can be swelling, skin discoloration and tissue necrosis. Though some injection site irritation is inevitable in some animals, injection site swelling of more than 2 x 2 cm that persists for more than two to three days is generally considered by veterinarians and animal owners to be unacceptable. Minimal injection site irritation means injection site irritation that is less than 2 x 2 cm that persists for less than two to three days. This standard is generally accepted by veterinarians and their clients in the context of animals receiving injections such as the rabies vaccine.

As used herein, particle size data reported are volume weighted as measured by conventional particle techniques well known to those skilled in the art, such as static light scattering (also known as laser diffraction), image analysis or sieving. More discussion of particle size measurement is provided below.

Pharmaceutically acceptable excipient is an inert substance that forms a vehicle or medium for a drug.

A parasite "infestation" refers to the presence of parasites in numbers that pose a risk to humans or animals. The presence can be in the environment, e.g., in animal bedding, on the skin or fur of an animal, etc. When the infestation that is referred to is within an animal, e.g., in the blood or other internal tissues, the term infestation is also intended to be synonymous with the term, "infection," as that term is generally understood in the art, unless otherwise stated.

Suspension means the state of a substance when its particles are mixed with but undissolved in a fluid (a liquid or a gas) or solid.

Diluent means the substance used to dilute a mixture, a suspension or a solution.

Vehicle is a carrier or inert medium used as a solvent (or diluent) in which the medicinally active agent is formulated and or administered.

The pharmaceutical compositions of the invention are of particular value in the control of ectoparasites, i.e. arthropods which are injurious to, or spread or act as vectors of diseases in man and livestock and companion animals.

Important arthropod parasites- ectoparasites (insect and acarid pests) are described below in more detail.

Biting insects include, e.g., migrating diperous larvae as *Hypoderma* sp. in cattle, *Gastrophilus* in horses, and *Cuterebra* sp. in rodents, as well as biting flies and mosquitoes spp of all types. For example, bloodsucking adult flies include, e.g., the horn fly or *Haematobia irritans*, the horse fly or *Tabanus* spp., the stable fly or *Stomoxys calcitrans,* the black fly or *Simulium* spp., the deer fly or *Chrysops* spp., the louse fly or *Melophagus ovinus,* the tsetse fly or *Glossina* spp. Parasitic fly maggots include, e.g., the bot fly (*Oestrus ovis* and *Cuterebra* spp.), the blow fly or *Phaenicia* spp., the screwworm or *Cochliomyia hominivorax,* the cattle grub or *Hypoderma* spp., and the fleeceworm. Mosquitos, include, for example, *Culex* spp., *Anopheles* spp., and *Aedes* spp.

Mites include the chicken mite, *Dermanyssus gallinae*; itch or scab mites or mange mites (*Astigmata*) such as *Sarcoptidae* spp. for example, *Sarcoptes scabiei;* mange mites such as *Psoroptidae* spp. including *Chorioptes bovis, Psoroptes ovis* and Demodex canis; the ear mite *Otodectes cynotis;* chiggers e.g. , *Trombiculidae* spp. for example the North American chigger, *Trombicula alfreddugesi.*

Ticks include, e.g., soft-bodied ticks including *Argasidae* spp. for example *Argas* spp. and *Ornithodoros* spp.; hard-bodied ticks including *Ixodidae* spp., for example *Ixodes ricinus, Ixodes scapularis, Rhipicephalus sanguineus, Haemaphysalis* spp, *Dermacentor reticulatus, Dermacentor variabilis, Amblyomma americanum* and *Boophilus* spp.

Lice include, e.g., sucking lice, e.g., *Menopon* spp. and *Bovicola* spp.; biting lice, e.g., *Haematopinus* spp., *Linognathus* spp. and *Solenopotes* spp.

Fleas include, e.g., *Ctenocephalides* spp., such as dog flea (*Ctenocephalides canis*) and cat flea (*Ctenocephalides felis*); *Xenopsylla* spp. such as oriental rat flea (*Xenopsylla cheopis*); and *Pulex* spp. such as human flea (*Pulex irritans*).

True bugs include, e.g., *Cimicidae* or e.g., the common bed bug (*Cimex lectularius*); *Triatominae* spp. including triatomid bugs also known as kissing bugs; for example *Rhodnius prolixus* and *Triatoma* spp.

The compositions of the invention are of value for the treatment and control of the various lifecycle stages of parasites including egg, nymph, and larvae, juvenile and adult stages.

For the avoidance of doubt, references herein to "treatment" as used herein includes references to curative and palliative treatment, references to "control of ectoparasites" include kill, repel, expel, incapacitate, deter, eliminate, alleviate, minimise, eradicate pests on animals and in the environment of animals.

"Control of ectoparasite infestation" means to alleviate or reduce parasite numbers in and/or on an animal, and/or to inhibit the development of parasite infestation in or on an animal, in whole or in part.

Prevention is stopping a new or incoming infestation or infection from establishing.

Control or "Efficacy" of a compound means that the parasite count is reduced, after a first administration, by an amount ranging from 5% to about 100%. The control of arthropods (e.g., insects, acarids) can be insecticidal, and/or acaricidal. The effect of the compounds of the invention can be e.g., ovicidal, larvicidal, nymphicidal and/or adulticidal or a combination thereof. The effect can manifest itself directly, i.e., killing the parasites either immediately or after some time has elapsed, for example when molting occurs, or by destroying their eggs, or indirectly, e.g., reducing the number of eggs laid and/or the hatching rate.

For an in vivo administration of the compound according to the invention, an effective amount is synonymous with a "pharmaceutically effective amount" which is the dose or amount that treats or ameliorates symptoms and/or signs of parasite infection or infestation by the treated animal or reduces parasite numbers in and/or on an animal, and/or to inhibits the development of parasite infestation in or on an animal, in whole or in part. This latter amount is also readily determined by one of ordinary skill in the art, e.g., by observing or detecting changes in clinical condition or behavior of treated animals, as well as by observing or detecting relative changes in parasite numbers after such treatment.

Systemic administration of medicaments means that the target (organ or parasite) is reached via the bloodstream.

Animal means mammals including companion animals. Companion animal means dog, cat or horse.

Reconstitutable formulation is a formulation where the vehicle is one container and the active ingredient is in another container and the two containers are combined at some point prior to administration.

Vehicle contains some or all of the excipients necessary for the formulation, for example the diluent, the wetting agent, the antifoaming agent, the ph control agent, etc.

In an embodiment, the isoxazoline compounds for use in the invention also include pharmaceutically acceptable salts, esters, and/or N-oxides thereof. In addition, the reference to an isoxazoline compound refers equally to any of its polymorphic forms or stereoisomers.

In an embodiment, the pharmaceutical composition according to the invention may employ a racemic mixture of an isoxazoline for use in the invention, containing equal amounts of the enantiomers of such isoxazoline compound as described above. Alternatively, the pharmaceutical composition may use isoxazoline compounds that contain enriched stereoisomers compared to the racemic mixture in one of the enantiomers of the isoxazoline as defined herein. Also, the pharmaceutical composition may use an essentially pure stereoisomer of such isoxazoline compounds. Such enriched- or purified stereoisomer preparations of an isoxazoline for use in the invention, may be prepared by methods known in the art. Examples are chemical processes utilizing catalytic asymmetric synthesis, or the separation of diastereomeric salts (see e.g.: WO 2009/063910, and JP 2011/051977, respectively).

In an embodiment of an isoxazoline for use in the invention, T is selected from 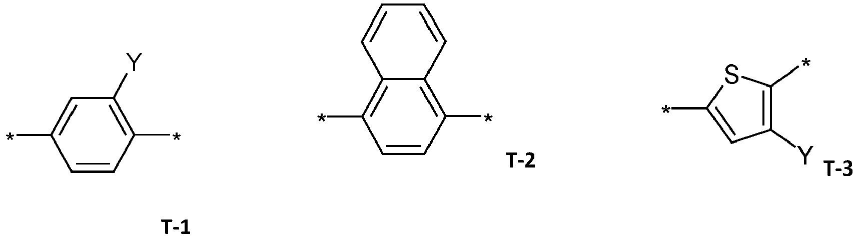 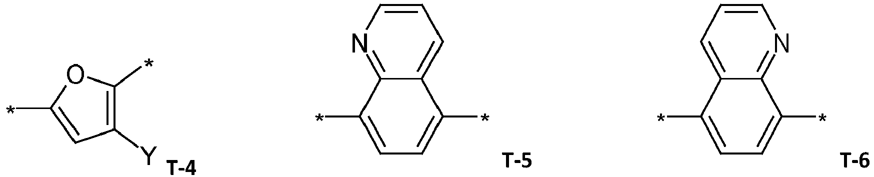 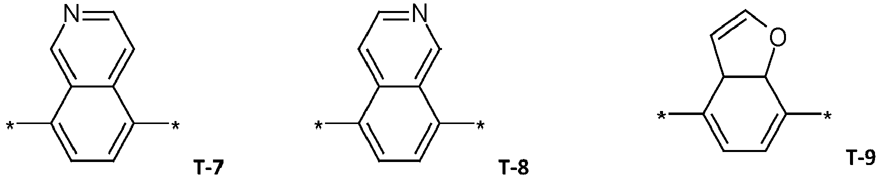 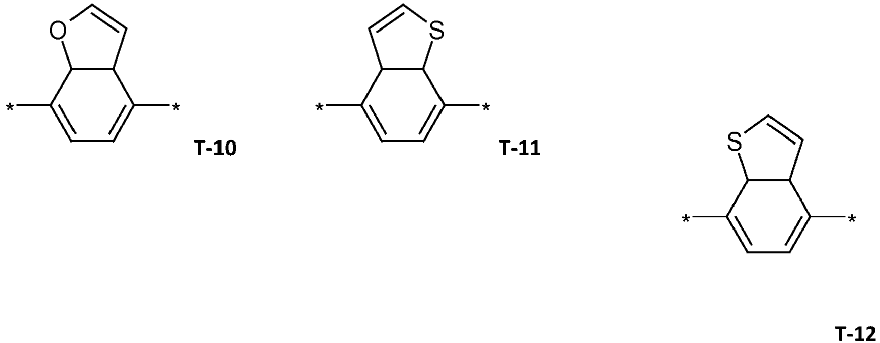 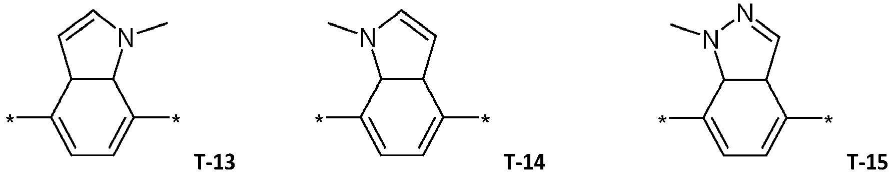 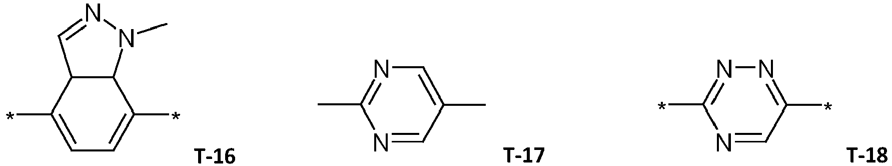 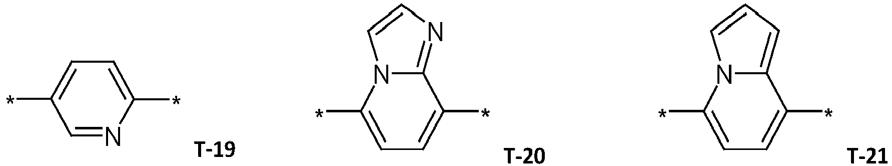 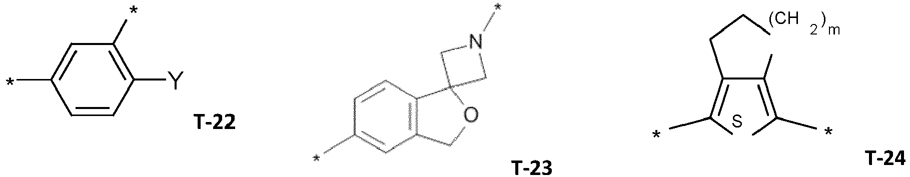 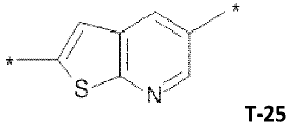 wherein in T-1, T-3 and T-4, the radical Y = hydrogen, halogen, methyl, halomethyl, ethyl, or haloethyl.

In an embodiment of an isoxazoline for use in the invention, Q is selected from 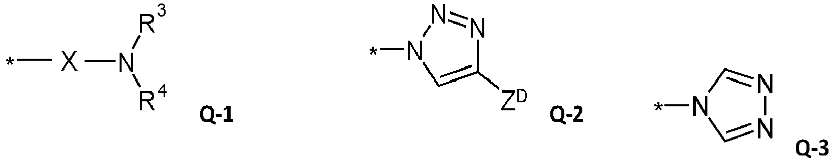 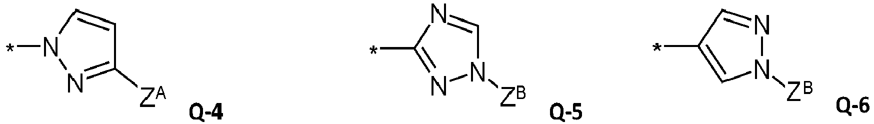 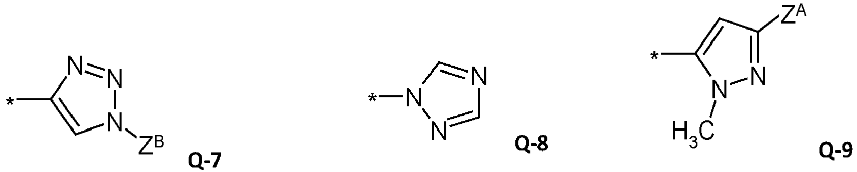
wherein R³, R⁴ , X and Z^{A} are as defined above, and
ZB = 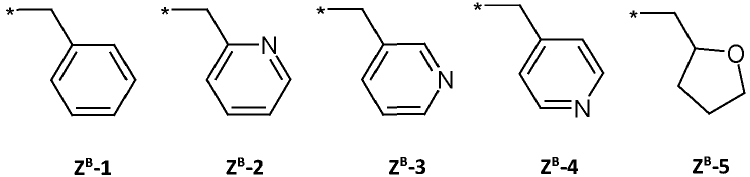 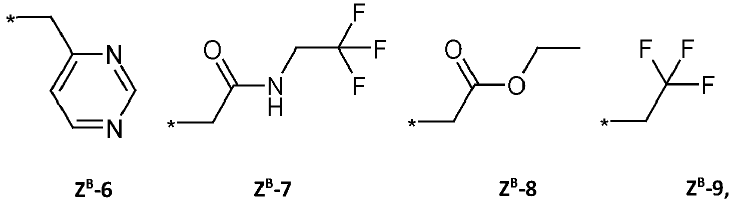
ZD =

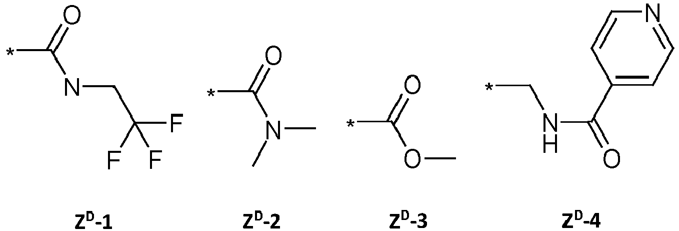

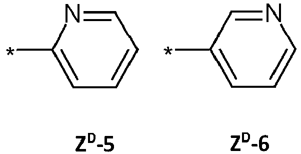

In an embodiment an isoxazoline for use in the invention is as presented in Table 1.

**Table 1:**

| **(R¹)ₙ** | **R²** | **R³** | **R⁴** | **T** | **Y** | **Q** | **Z** | **X** |
|---|---|---|---|---|---|---|---|---|
| 3-Cl, 5-Cl | CF₃ | CH₂CF₃ | H | T-2 | - | Q-1 | - | CO |
| 3-Cl, 5-Cl | CF₃ | CH₂CH₃ | H | T-2 | - | Q-1 | - | CO |
| 3-Cl, 5-Cl | CF₃ | CH₂CH₂OCH₃ | H | T-2 | - | Q-1 | - | CO |
| 3-Cl, 5-Cl | CF₃ | CH₂C(O)NHCH₂CF₃ | H | T-2 | - | Q-1 | - | CO |
| 3-CI, 5-Cl | CF₃ | CH₂C(O)NHCH₂CH₃ | H | T-2 | - | Q-1 | - | CO |
| 3-CF₃, 5-CF₃ | CF₃ | CH₂C(O)NHCH₂CF₃ | H | T-2 | - | Q-1 | - | CO |
| 3-CF₃, 5-CF₃ | CF₃ | CH₂C(O)NHCH₂CH₃ | H | T-2 | - | Q-1 | - | CO |
| 3-CF₃, 5-Cl | CF₃ | CH₂C(O)NHCH₂CF₃ | H | T-2 | - | Q-1 | - | CO |
| 3-CF₃, 5-Cl | CF₃ | CH₂C(O)NHCH₂CH₃ | H | T-2 | - | Q-1 | - | CO |
| 3-Cl, 5-Cl | CF₃ | - | | T-2 | - | Q-6 | Z^{B}-7 | CO |
| 3-Cl, 5-Cl | CF₃ | - | - | T-2 | - | Q-7 | Z^{B}-7 | CO |
| 3-Cl, 5-Cl | CF₃ | - | - | T-2 | - | Q-5 | Z^{B}-7 | CO |
| 3-Cl, 5-Cl | CF₃ | - | - | T-2 | - | Q-2 | Z^{D}-1 | CO |
| 3-Cl, 5-Cl | CF₃ | CH₂C(O)NHCH₂CF₃ | H | T-3 | CH₃ | Q-1 | - | CO |
| 3-Cl, 5-Cl | CF₃ | CH₂C(O)NHCH₂CC | H | T-3 | CH₃ | Q-1 | - | CO |
| 3-Cl, 5-Cl | CF₃ | CH₂C(O)NHCH₂CN | H | T-3 | CH₃ | Q-1 | - | CO |
| 3-Cl, 5-Cl | CF₃ | CH₂C(O)NHCH₂CH₃ | H | T-3 | CH₃ | Q-1 | - | CO |
| 3-CF₃, 5-CF₃ | CF₃ | CH₂C(O)NHCH₂CF₃ | H | T-3 | CH₃ | Q-1 | - | CO |
| 3-CF₃, 5-CF₃ | CF₃ | CH₂C(O)NHCH₂CH₃ | H | T-3 | CH₃ | Q-1 | - | CO |
| 3-Cl, 4-Cl, 5-Cl | CF₃ | CH₂C(O)NHCH₂CF₃ | H | T-3 | CH₃ | Q-1 | - | CO |
| 3-Cl, 4-Cl, 5-Cl | CF₃ | CH₂C(O)NHCH₂CH₃ | H | T-3 | CH₃ | Q-1 | - | CO |
| 3-Cl, 4-F, 5-Cl | CF₃ | CH₂C(O)NHCH₂CF₃ | H | T-3 | CH₃ | Q-1 | - | CO |
| 3-Cl, 4-F, 5-Cl | CF₃ | CH₂C(O)NHCH₂CH₃ | H | T-3 | CH₃ | Q-1 | - | CO |
| 3-Cl, 5-Cl | CF₃ | CH₂C(O)NHCH₂CF₃ | H | T-20 | - | Q-1 | - | CO |
| 3-Cl, 5-Cl | CF₃ | CH₂C(O)NHCH₂CH₃ | H | T-20 | - | Q-1 | - | CO |
| 3-CF₃, 5-CF₃ | CF₃ | CH₂C(O)NHCH₂CF₃ | CH₃ | T-20 | - | Q-1 | - | CO |
| 3-CF₃, 5-CF₃ | CF₃ | CH₂C(O)NHCH₂CH₃ | CH₃ | T-20 | - | Q-1 | - | CO |
| 3-CF₃, 5-CF₃ | CF₃ | CH₂C(O)NHCH₂CF₃ | H | T-20 | - | Q-1 | - | CO |
| 3-CF₃, 5-CF₃ | CF₃ | CH₂C(O)NHCH₂CH₃ | H | T-20 | - | Q-1 | - | CO |
| 3-CF₃, 5-CF₃ | CF₃ | CH₂C(O)NHCH₂CF₃ | H | T-21 | - | Q-1 | - | CO |
| 3-CF₃, 5-CF₃ | CF₃ | CH₂C(O)NHCH₂CH₃ | H | T-21 | - | Q-1 | - | CO |
| 3-Cl, 5-Cl | CF₃ | CH₂C(O)NHCH₂CF₃ | H | T-21 | - | Q-1 | - | CO |
| 3-Cl, 5-Cl | CF₃ | CH₂C(O)NHCH₂CH₃ | H | T-21 | - | Q-1 | - | CO |
| 3-Cl, 5-Cl | CF₃ | CH₂CH₂SCH₃ | H | T-21 | - | Q-1 | - | CO |
| 3-Cl, 4-Cl, 5-Cl | CF₃ | C(O)CH₃ | H | T-22 | F | Q-1 | - | CH₂ |
| 3-Cl, 4-Cl, 5-Cl | CF₃ | C(O)CH(CH₃)₂ | H | T-22 | F | Q-1 | - | CH₂ |
| 3-Cl, 4-Cl, 5-Cl | CF₃ | C(O)-cyclo-propyl | H | T-22 | F | Q-1 | - | CH₂ |
| 3-Cl, 4-F, 5-Cl | CF₃ | C(O)CH₃ | H | T-22 | F | Q-1 | - | CH₂ |
| 3-Cl, 4-Cl, 5-Cl | CF₃ | C(O)CH₂CH₃ | H | T-22 | F | Q-1 | - | CH₂ |
| 3-Cl, 4-F, 5-Cl | CF₃ | C(O)CH₃ | H | T-22 | Cl | Q-1 | - | CH₂ |
| 3-Cl, 5-Cl | CF₃ | CH₂C(O)NHCH₂CF₃ | H | T-1 | CH₃ | Q-1 | - | CO |
| 3-Cl, 5-Cl | CF₃ | CH₂C(O)NHCH₂CH₃ | H | T-1 | CH₃ | Q-1 | - | CO |
| 3-Cl, 5-Cl | CF₃ | R³-1 (Z) | H | T-1 | CH₃ | Q-1 | - | CO |
| 3-Cl, 5-Cl | CF₃ | R³-1 (E) | H | T-1 | CH₃ | Q-1 | - | CO |

In an embodiment an isoxazoline for use in the invention is as presented in Table 2.

**Table 2:**

| **(R¹)ₙ** | **R²** | **R³** | **R⁴** | **T** | **Y** | **Q** | **Z** | **X** |
|---|---|---|---|---|---|---|---|---|
| 3-Cl, 5-Cl | CF₃ | CH₂CF₃ | H | T-2 | - | Q-1 | - | CO |
| 3-Cl, 5-Cl | CF₃ | CH₂CH₃ | H | T-2 | - | Q-1 | - | CO |
| 3-Cl, 5-Cl | CF₃ | CH₂CH₂OCH₃ | H | T-2 | - | Q-1 | - | CO |
| 3-Cl, 5-Cl | CF₃ | CH₂C(O)NHCH₂CF₃ | H | T-2 | - | Q-1 | - | CO |
| 3-CF₃, 5-CF₃ | CF₃ | CH₂C(O)NHCH₂CF₃ | H | T-2 | - | Q-1 | - | CO |
| 3-CF₃, 5-Cl | CF₃ | CH₂C(O)NHCH₂CF₃ | H | T-2 | - | Q-1 | - | CO |
| 3-Cl, 5-Cl | CF₃ | - | | T-2 | - | Q-6 | Z^{B}-7 | |
| 3-Cl, 5-Cl | CF₃ | - | - | T-2 | - | Q-7 | Z^{B}-7 | |
| 3-Cl, 5-Cl | CF₃ | - | - | T-2 | - | Q-5 | Z^{B}-7 | |
| 3-Cl, 5-Cl | CF₃ | - | - | T-2 | - | Q-2 | Z^{D}-1 | |
| 3-Cl, 5-Cl | CF₃ | CH₂C(O)NHCH₂CF₃ | H | T-3 | CH₃ | Q-1 | - | CO |
| 3-Cl, 5-Cl | CF₃ | CH₂C(O)NHCH₂CC | H | T-3 | CH₃ | Q-1 | - | CO |
| 3-Cl, 5-Cl | CF₃ | CH₂C(O)NHCH₂CN | H | T-3 | CH₃ | Q-1 | - | CO |
| 3-CF₃, 5-CF₃ | CF₃ | CH₂C(O)NHCH₂CF₃ | H | T-3 | CH₃ | Q-1 | - | CO |
| 3-Cl, 4-Cl, 5-Cl | CF₃ | CH₂C(O)NHCH₂CF₃ | H | T-3 | CH₃ | Q-1 | - | CO |
| 3-Cl, 4-F, 5-Cl | CF₃ | CH₂C(O)NHCH₂CF₃ | H | T-3 | CH₃ | Q-1 | - | CO |
| 3-Cl, 5-Cl | CF₃ | CH₂C(O)NHCH₂CF₃ | H | T-20 | - | Q-1 | - | CO |
| 3-CF₃, 5-CF₃ | CF₃ | CH₂C(O)NHCH₂CF₃ | CH₃ | T-20 | - | Q-1 | - | CO |
| 3-CF₃, 5-CF₃ | CF₃ | CH₂C(O)NHCH₂CF₃ | H | T-20 | - | Q-1 | - | CO |
| 3-CF₃, 5-CF₃ | CF₃ | CH₂C(O)NHCH₂CF₃ | H | T-21 | - | Q-1 | - | CO |
| 3-Cl, 5-Cl | CF₃ | CH₂C(O)NHCH₂CF₃ | H | T-21 | - | Q-1 | - | CO |
| 3-Cl, 5-Cl | CF₃ | CH₂CH₂SCH₃ | H | T-21 | - | Q-1 | - | CO |
| 3-Cl, 4-Cl, 5-Cl | CF₃ | C(O)CH₃ | H | T-22 | F | Q-1 | - | CH₂ |
| 3-Cl, 4-Cl, 5-Cl | CF₃ | C(O)CH(CH₃)₂ | H | T-22 | F | Q-1 | - | CH₂ |
| 3-Cl, 4-Cl, 5-Cl | CF₃ | C(O)-cyclo-propyl | H | T-22 | F | Q-1 | - | CH₂ |
| 3-Cl, 4-F, 5-Cl | CF₃ | C(O)CH₃ | H | T-22 | F | Q-1 | - | CH₂ |
| 3-Cl, 4-Cl, 5-Cl | CF₃ | C(O)CH₂CH₃ | H | T-22 | F | Q-1 | - | CH₂ |
| 3-Cl, 4-F, 5-Cl | CF₃ | C(O)CH₃ | H | T-22 | Cl | Q-1 | - | CH₂ |
| 3-Cl, 5-Cl | CF₃ | CH₂C(O)NHCH₂CF₃ | H | T-1 | CH₃ | Q-1 | - | CO |
| 3-Cl, 5-Cl | CF₃ | R³-1(Z) | H | T-1 | CH₃ | Q-1 | - | CO |
| 3-Cl, 5-Cl | CF₃ | R³-1(E) | H | T-1 | CH₃ | Q-1 | - | CO |

In an embodiment an isoxazoline for use in the invention is the compound: 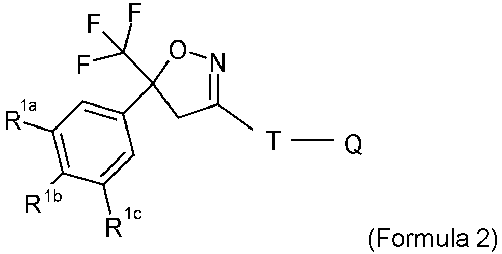 wherein R^{1a}, R^{1b}, R^{1c} are independently from each other: hydrogen, Cl or CF₃.

Preferably R^{1a} and R^{1c} are Cl or CF₃, and R^{1b} is hydrogen,
T is 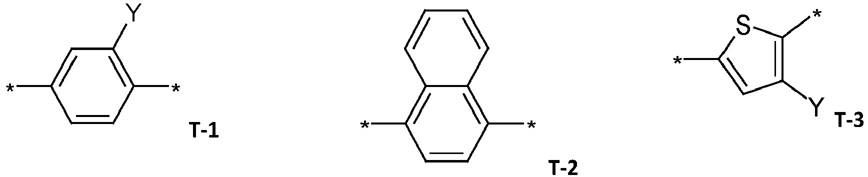 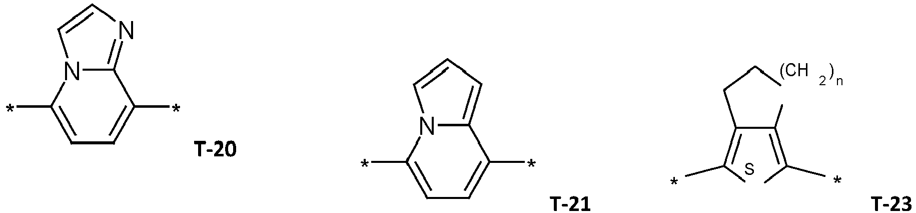 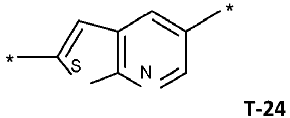
wherein Y is methyl, bromine, Cl, F, CN or C(S)NH₂; n = 1 or 2; and Q is as described above.

In an embodiment of an isoxazoline as defined herein, R³ is H, and R⁴ is: -CH₂-C(O)-NH-CH₂-CF₃, -CH₂-C(O)-NH-CH₂-CH₃, -CH₂-CH₂-CF₃ or -CH₂-CF_{3.}

In an embodiment of the pharmaceutical composition according to the invention, the isoxazoline is one or more selected from the group consisting of fluralaner, afoxolaner, lotilaner or sarolaner.

In one embodiment the compound of Formula (I) is 4-[5-(3,5-Dichlorophenyl)-5-trifluoromethyl-4,5-dihydroisoxazol-3-yl]-2-methyl-*N*-[(2,2,2-trifluoro-ethylcarbamoyl)-methyl]-benzamide (CAS RN 864731-61-3 - USAN fluralaner).

In another embodiment the compound of Formula (I) is 4-[5-[3-Chloro-5-(trifluoromethyl)phenyl]-4,5-dihydro-5-(trifluoromethyl)-3-isoxazolyl]-N-[2-oxo-2-[(2,2,2-trifluoroethyl)amino]ethyl]-1-naphthalenecarboxamide (CAS RN 1093861-60-9, USAN - afoxolaner) that was disclosed in WO2007/079162-.

In an embodiment of the pharmaceutical composition according to the invention the isoxazoline is lotilaner (CAS RN: 1369852-71-0; 3-methyl-N-[2-oxo-2-(2,2,2-trifluoroethylamino)ethyl]-5-[(5S)-5-(3,4,5-trichlorophenyl)-5-(trifluoromethyl)-4H-1,2-oxazol-3-yl]thiophene-2-carboxamide).

In an embodiment of the pharmaceutical composition according to the invention the isoxazoline is sarolaner (CAS RN: 1398609-39-6; 1-(5'-((5S)-5-(3,5-dichloro-4-fluorophenyl)-5-(trifluoromethyl)-4,5-dihydroisoxazol-3-yl)-3'-H-spiro(azetidine-3,1'-(2) benzofuran)-1-yl)-2-(methylsulfonyl) ethanone).

In another embodiment the compound of Formula (I) is (Z)-4-[5-(3,5-Dichlorophenyl)-5-trifluoromethyl-4,5-dihydroisoxazol-3-yl]-N-[(methoxyimino)methyl]-2-methylbenzamide (CAS RN 928789-76-8).

In another embodiment the compound of Formula (I) is 4-[5-(3,5-dichlorophenyl)-5-(trifluoromethyl)-4H-isoxazol-3-yl]-2-methyl-N-(thietan-3-yl)benzamide (CAS RN 1164267-94-0) that was disclosed in WO2009/0080250.

In another embodiment the compound of Formula (I) is 4-[5-[3-Chloro-5-(trifluoromethyl)phenyl]-4,5-dihydro-5-(trifluoromethyl)-3-isoxazolyl]-N-[2-oxo-2-[(2,2,2-trifluoroethyl)amino]ethyl]-1-naphthalenecarboxamide (CAS RN 1093861-60-9, USAN - afoxolaner) that was disclosed in WO2007/079162-.

In another embodiment the compound of Formula (I) is 5-[5-(3,5-Dichlorophenyl)-4,5-dihydro-5-(trifluoromethyl)-3-isoxazolyl]-3-methyl-N-[2-oxo-2-[(2,2,2-trifluoroethyl)amino]ethyl]- 2-thiophenecarboxamide (CAS RN 1231754-09-8) that was disclosed in WO2010/070068.

The long-acting injectable compositions of the invention include pharmaceutically acceptable excipients. Pharmaceutically acceptable excipients include, but are not limited to, surfactants, antioxidants, preservatives, pH stabilizing agents (e.g. buffers), and other non-active excipients. In another embodiment, the compositions of the invention may comprise about 0.01% to about 20% (w/v) of pharmaceutically acceptable excipients. In other embodiments, the compositions may comprise about 0.01% to about 5% (w/v), about 0.1% to about 10% (w/v) or about 0.1% to about 5% (w/v) of pharmaceutically acceptable excipients. In other embodiments the compositions may comprise about 5 to about 15% (w/v) or about 5 to about 10% (w/v) of pharmaceutically acceptable excipients. In yet another embodiment, the compositions may comprise about 7 to about 10% of pharmaceutically acceptable excipients.

Surfactants may be present in the inventive compositions at concentrations of about 0.1% to about 10% (w/w), about 1% to about 10% (w/w) or about 5% to about 10% (w/w). More typically, surfactants may be present at concentrations of about 0.1% to about 5% (w/w) or about 1 to about 5% (w/w). Examples of surfactants that may be used in the compositions include, but are not limited to, glyceryl monooleate, polyoxyethylene sorbitan fatty acid esters, sorbitan esters including sorbitan monooleate (Span^{®} 20), polyvinyl alcohol, polysorbates including polysorbate 20 and polysorbate 80, d-a-tocopherol polyethylene glycol 1000 succinate (TPGS), sodium lauryl sulfate, co-polymers of ethylene oxide and propylene oxide (e.g. poloxamers such as LUTROL^{®} F87 and the like), polyethylene glycol castor oil derivatives including polyoxyl 35 castor oil (Cremophor^{®} EL), polyoxyl 40 hydrogenated castor oil (Cremophor^{®} RH 40), polyoxyl 60 hydrogenated castor oil (Cremophor^{®} RH60); propylene glycol monolaurate (LAUROGLYCOL^{®}); glyceride esters including glycerol caprylate/caprate (CAPMULO MCM), polyglycolized glycerides)(GELUCIRE^{®}, PEG 300 caprylic/capric glycerides (Softigen^{®} 767), PEG 400 caprylic/capric glycerides (Labrasol^{®}), PEG 300 oleic glycerides (Labrafil^{®} M-1944CS), PEG 300 linoleic glycerides (Labrafil^{®} M-2125CS); polyethylene glycol stearates and polyethylene glycol hydroxy stearates including polyoxyl 8 stearate (PEG 400 monostearate), polyoxyl 40 stearate (PEG 1750 monostearate, and the like). Polyethylene glycol stearates (synonyms include macrogol stearates, polyoxylstearates, polyoxyethylene stearates, ethoxylated stearates; CAS No. 9004-99-3, 9005-08-7) are mixtures of mono- and distearate esters of mixed polyoxyethylene polymers. Polyethylene glycol hydroxystearate is a mixture of mono-and diesters of hydroxystearic acid with polyethylene glycols. One polyethylene glycol hydroxystearate that may be used in the compositions is polyethylene glycol 12-hydroxystearate. In another embodiment, the inventive compositions may include the surfactant polyethylene glycol 15 12-hydroxystearate (Kolliphor^{®} HS 15 from BASF), a mixture of mono- and diesters of 12-hydroxystearic acid with 15 moles of ethylene oxide. Again, these compounds, as well as their amounts are well known in the art. In another embodiment of the invention, the inventive compositions may include polyoxyl 35 castor oil (Kolliphor^{®} EL) as a surfactant. In other embodiments, the inventive compositions may include polyoxyl 40 hydrogenated castor oil (Kolliphor^{®} RH 40) or polyoxyl 60 hydrogenated castor oil as surfactants. The compositions of the invention may also include a combination of surfactants.

The inventive compositions may contain other inert ingredients such as antioxidants, preservatives, or pH stabilizers. These compounds are well known in the composition art. Antioxidants such as vitamin E, alpha tocopherol, ascorbic acid, ascorbyl palmitate, citric acid, fumaric acid, malic acid, sodium ascorbate, sodium metabisulfate, sodium metabisulfite, n-propyl gallate, BHA (butylated hydroxy anisole), BHT (butylated hydroxy toluene), BHA and citric acid, monothioglycerol, tert-butyl hydroquinone (TBHQ), benzyl alcohol and the like, may be added to the present composition. The antioxidants are generally included in the compositions of the invention in amounts of about 0.01% to about 3%, or from about 0.01 to about 2% (w/v), based upon total weight of the composition (w/w). In another embodiment, the compositions contain about 0.05 to about 1.0% (w/w) of one or a mixture of antioxidants.

Preservatives, such as benzyl alcohol, are suitably used in the composition in amounts ranging from about 0.01 to about 10.0%, with about 0.05 to about 5.0% being especially preferred. Other preservatives include parabens (methylparaben and/or propylparaben), benzalkonium chloride, benzethonium chloride, benzoic acid, benzyl alcohol, bronopol, butylparaben, cetrimide, chlorhexidine, chlorobutanol, chlorocresol, cresol, ethylparaben, imidurea, methylparaben, phenol, phenoxyethanol, phenylethyl alcohol, phenylmercuric acetate, phenylmercuric borate, phenylmercuric nitrate, potassium sorbate, sodium benzoate, sodium propionate, sorbic acid, thimerosal, and the like. Preferred ranges for these compounds include from about 0.01 to about 5%.

Compounds which stabilize the pH of the composition may also be present. Again, such compounds are well known to a practitioner in the art as well as how to use these compounds. Buffering systems include, for example, systems selected from the group consisting of acetic acid/acetate, malic acid/malate, citric acid/citrate, tartaric acid/tartrate, lactic acid/lactate, phosphoric acid/phosphate, glycine/glycimate, tris, glutamic acid/glutamates and sodium carbonate, especially sodium phosphate or sodium citrate.

Oily suspensions (non-aqueous suspensions) may be formulated by suspending the isoxazoline compound in a vegetable oil, for example, arachis oil, olive oil, sesame oil or coconut oil, or in mineral oil such as liquid paraffin or other pharmaceutically acceptable oils. The oily suspensions may contain a thickening agent, for example, beeswax, hard paraffin or cetyl alcohol. These compositions may be preserved by the addition of an anti-oxidant such as ascorbic acid, or other known preservatives.

In yet another embodiment of the invention, the oily suspension injectable compositions of the invention include non-water miscible co-solvents. Non-limiting examples of these co-solvents include benzyl benzoate, ethyl acetate, triacetin, lipids, triglycerides including medium chain triglycerides such C8-C10 triglycerides such as capric/caprilic triglycerides, propylene glycol derivatives (e.g. propylene glycol monolaurate), caprylocaproyl polyoxyl-8 glycerides (Labrasol) (non-ionic water dispersible surfactant, isopropyl myristate, or a mixture of at least two of these co-solvents.

In another embodiment, the non-aqueous injectable composition of the invention may include neutral oils as a co-solvent. Neutral oils are triglycerides of fractionated plant fatty acids with chain lengths of C8 to C10. Two commercially available products are known as MIGLYOLO 810 and MIGLYOLO 812. In another embodiment, the neutral oil is a triglyceride of fractionated plant fatty acids with chain lengths of C8 and C10 combined with linoleic acid (about 4-5%). A commercially available product is known as MIGLYOLO 818. In yet another embodiment, the neutral oil is a glycerin ester of fractionated plant fatty acids with chain lengths of C8 and C10 combined with succinic acid. A commercially available product is known as MIGLYOLO 829. In another embodiment, the neutral oil may be a propylene glycol diester of saturated plant fatty acids with chain lengths of C8 and C10. A commercially available product is known as MIGLYOLO 840 (propylene glycol dicaprylate/dicaprate). In yet another embodiment, the co-solvent may be a mixture of two or more neutral oils.

Aqueous suspensions may contain the isoxazoline compound in admixture with excipients suitable for the manufacture of aqueous suspensions. Such excipients include suspending agents, for example, sodium carboxymethylcellulose, methylcellulose, hydroxy-propylmethylcellulose, sodium alginate, polvinylpyrrolidone, gum tragacanth and gum acacia; dispersing or wetting agents include naturally-occurring phosphatide, for example lecithin, or condensation products of an alkylene oxide with fatty acids, for example polyoxyethylene stearate, or condensation products of ethylene oxide with long chain aliphatic alcohols, for example, heptadecaethyleneoxycetanol, or condensation products of ethylene oxide with partial esters derived from fatty acids and a hexitol such as polyoxyethylene sorbitol monooleate, or condensation products of ethylene oxide, with partial esters derived from fatty acids and hexitol anhydrides, for example polyethylene sorbitan monooleate. The aqueous suspensions may also contain one or more preservatives, for example ethyl, or n-propyl, p-hydroxybenzoate, one or more coloring agents, one or more flavoring agents, and one or more sweetening agents and/or bittering agents, such as those set forth above.

Dispersible powders and granules suitable for preparation of an aqueous suspension by the addition of water may provide the isoxazoline compound in admixture with a dispersing or wetting agent, suspending agent and one or more preservatives. Suitable dispersing or wetting agents and suspending agents are exemplified by those already mentioned above.

In one embodiment the isoxazoline compound is suspended in an aqueous suspension wherein the liquid phase (diluent) is water.

In another embodiment the liquid phase (diluent) of the aqueous suspension comprises water and a co-solvent.

Co-solvents that might be used in the inventive injectable compositions comprising a isoxazoline compound may be a single or a blend of co-solvents.

In one embodiment, the co-solvents used in the aqueous injectable compositions of the present invention include polar solvents that are miscible in water. Non-limiting examples of these co-solvents include ethanol, isopropanol, benzyl alcohol, glycol ethers (e.g., including, but limited to, diethyleneglycol monoethyl ether (DGME, Transcutol^{®}, butyl diglycol, dipropylene glycol n-butyl ether, ethyleneglycol monoethyl ether, ethyleneglycol monomethyl ether, dipropylene glycol monomethyl ether, propylene glycol monomethyl ether, propylene glycol monoethyl ether, and the like), liquid polyethylene glycols (PEGs) (for example, PEG 400), propylene glycol, carbonates (e.g., propylene carbonate), 2-pyrrolidone, N-methylpyrrolidone, dimethyl isosorbide (DMI), dimethylacetamide, dimethylsulfoxide, glycerol formal or a mixture of at least two of these solvents.

In one embodiment, the compositions of the invention comprise a polar protic solvent including, but not limited to, an alcohol such as ethanol, isopropanol or a glycol or glycol ether. In another embodiment, the long-acting injectable compositions of the invention comprise a polar aprotic solvent such as N-methylpyrrolidone, dimethyl isosorbide, dimethylacetamide, dimethylsulfoxide or propylene carbonate.

In an embodiment, the isoxazoline compounds may exist in various isomeric forms. A reference to an isoxazoline compound always includes all possible isomeric forms of such compound. Unless otherwise stated, a compound structure that does not indicate a particular conformation is intended to encompass compositions of all the possible conformational isomers of the compound, as well as compositions comprising fewer than all the possible conformational isomers. In some embodiments, the compound is a chiral compound. In some embodiments, the compound is a non-chiral compound.

In an embodiment, the isoxazoline compounds of Formula (I) can be prepared according to one or other of the processes described e.g. in Patent Applications US 2007/0066617, WO 2007/079162, WO 2009/002809, WO 2009/080250, WO 2010/070068, WO 2010/079077, 2011/075591 and WO 2011/124998 or any other process coming within the competence of a person skilled in the art who is an expert in chemical synthesis. For the chemical preparation of the products of the invention, a person skilled in the art is regarded as having at his disposal, inter alia, the entire contents of "Chemical Abstracts" and of the documents which are cited therein.

In an embodiment, the isoxazoline compound is in suspension in the composition. In an embodiment, the suspension is aqueous. In an alternative embodiment, the suspension is non-aqueous.

In an embodiment, the pharmaceutical composition is substantially organic solvent free.

In an embodiment, the pharmaceutical composition comprises a surfactant/wetting agent. In another embodiment, the surfactant/wetting agent is poloxamer. Alternatives to the poloxamer are other water soluble/miscible non-ionic surfactants including sorbitan fatty acid esters (Spans), polyoxyethylene sorbitan fatty acid esters (polysorbateslTweens), polyoxyethylene castor oil derivatives (Cremaphors), polyoxyethylene stearates, lecithin and TPGS (d-α-Tocopheryl polyethylene glycol 1000 succinate). The surfactant/wetting agent is present in the composition in an amount of about 0.01 % w/v to about 0.5% w/v or about 0.05 % w/v to about 0.1% w/v.

Poloxamers are nonionic triblock copolymers composed of a central hydrophobic chain of polyoxypropylene (polypropylene oxide)) flanked by two hydrophilic chains of polyoxyethylene (poly(ethylene oxide)) (see U.S. Patent No. 3,740,421).

Poloxamer 124 is poly(ethylene glycol)-block-poly(propylene glycol)-block-poly(ethylene glycol, CAS Number 9003-11-6. Also known as Lutrol L44 or Kollisolv P124.

Lutrol F68 is another poly(ethylene glycol)-*block*-poly(propylene glycol)-*block-*poly(ethylene glycol), Also known as Poloxamer 188 or Kolliphor P188.

Lecithins
aremixtures glycerophospholipids including phosphatidylcholine, phosphatidylethanola mine, phosphatidylinositol, and phosphatidic acid.

In an embodiment, the pharmaceutical composition comprises a suspending agent. In an embodiment, the suspending agent is sodium carboxy methyl cellulose (NaCMC). In an alternative embodiment, the suspending agent is methylcellulose or polyvinyl pyrrolidone.

In an embodiment, the pharmaceutical composition comprises a anti-foaming agent. In an embodiment, the antifoaming agent is simeticone. Simeticone is α-(trimethylsilyl)-ω-methylpoly[oxy(dimethylsilylene)] mixture with silicon dioxide.

In an embodiment, the pharmaceutical composition comprises a preservative. In an embodiment, the preservative is benzyl alcohol. In an alternative embodiment, the preservative is m-cresol, benzalkonium chloride, methylparaben, or propylparaben.

The injectable pharmaceutical compositions may be made by combining and mixing the solid components and then suspending the solid mixture in the diluent.

The method of preparing the injectable pharmaceutical composition comprising combining isoxazoline particles with a wetting agent, and a diluent.

In an embodiment, the pharmaceutical composition is a reconstitutable solid, which is reconstituted with a diluent prior to injection.

In an embodiment, the diluent is water. In an alternative embodiment, the diluent is an oil or a solvent with little or no solubility for the isoxazoline compound.

The pharmaceutical composition further comprises a surfactant/wetting agent. Specific surfactants/ wetting agents and alternatives for the surfactant/wetting agent are discussed in this specification and in the Examples.

The pharmaceutical composition further comprises additional excipients such as a suspending agent, an anti-foaming agent or a preservative. Specific examples of suitable excipients and alternatives agent are discussed in this specification below and in the Examples.

### Isoxazoline compound particle size and measurement

It has been found that the inventive injectable compositions comprising particles of isoxazoline compounds with a defined particle size have especially beneficial properties.

In an embodiment, the isoxazoline compound has a particle size distribution of D50 as measured by a static light scattering instrument of from about 25 microns to about 250 microns, particle size of from about 11 microns to about 250 microns, particle size of from about 50 microns to about 150 microns, particle size of from about 75 microns to about 130 microns, particle size of from about 90 microns to about 110 microns. particle size of from about 30 microns to about 100 microns.

Particle size distribution describes the relative amount of particles present according to size. D10 is a particle size distribution that expresses the size that 10 % of the particles are smaller than. D50 is a particle size measurement distribution that expresses the size that 50 % of the particles are smaller than. D90 is a particle size measurement distribution that expresses the size that 90 % of the particles are smaller than.

In a particular embodiment, the D10 of particle size is about 10 µm, about 20 µm, about 30 µm, about 40 µm, about 50 µm, about 60 µm, or about 80 µm.

In a particular embodiment, the D50 of particle size is about 50 µm, about 75 µm, about 80 µm, about 90 µm, about 100 µm, about 110 µm, about 120 µm, about 130 µm, about 140 µm or about 150 µm.

In a particular embodiment, the D90 of particle size is about 100 µm, about 130 µm, about 150 µm, about 175 µm, about 200 µm, or about 250 µm.

In a particular embodiment, the D10 of the particle size is about 20 to 35 µm, the D50 of the particle size is about 90 to 105 µm and the D90 of the particle size is about 155 to 175 µm.

In a particular embodiment, the D10 of the particle size is about 25 to 30 µm, the D50 of the particle size is about 95 to 100 µm and the D90 of the particle size is about 160 to 170 µm.

In a particular embodiment, the D10 of the particle size is about 10 to 20 µm, the D50 of the particle size is about 85 to 110 µm and the D90 of the particle size is about 170 to 185 µm.

In a particular embodiment, the D10 of the particle size is about 10 to 15 µm, the D50 of the particle size is about 95 to 105 µm and the D90 of the particle size is about 175 to 180 µm.

In a particular embodiment, the D10 of the particle size is about 10 to 25 µm, the D50 of the particle size is about 40 to 60 µm and the D90 of the particle size is about 95 to 100 µm.

In a particular embodiment, the D10 of the particle size is about 15 to 20 µm, the D50 of the particle size is about 45 to 55 µm and the D90 of the particle size is about 90 to 95 µm.

In a particular embodiment, the D10 of the particle size is about 30 to 50 µm and the D50 of the particle size is about 70 to 130 µm.

In a particular embodiment, the D10 of the particle size is about 35 to 45 µm and the D50 of the particle size is about 90 to 110 µm.

In a particular embodiment, the D10 of the particle size is about 40 µm and the D50 of the particle size is about 100 µm.

The volume weighted particle size can be measured by sieving, microscopy or laser diffraction (Malvern or Sympatec) The volume weighted particle size measurement can be performed with a Malvern Mastersizer 2000 with the Hydro 2000G measuring cell, or with a Horiba LA-910 laser scattering particle size distribution analyzer. The volume weighted particle size can be measured by a Sympatec Helos instrument.

For use in the invention, the isoxazoline compound is present in the pharmaceutical composition according to the invention in an amount of between about 0.1 and about 50 % w/v of the final pharmaceutical composition according to the invention. The isoxazoline is present in an amount of between about 10 and about 45 % w/v; about 20 and about 45 % w/v; about 15 and 35 % w/v or about 25% w/v and about 35 % w/v of or about 1% w/v and about 12 % w/v of or about 3% w/v and about 9 % w/v the pharmaceutical composition according to the invention.

In an embodiment, the amount of isoxazoline compound in the pharmaceutical composition according to the invention is about 30 % w/v of the pharmaceutical composition according to the invention. In an embodiment, the amount of isoxazoline compound in the pharmaceutical composition according to the invention is about 7.5 % w/v of the pharmaceutical composition according to the invention.

In an embodiment, the pharmaceutical composition is a ready to use composition. That is the composition is ready for injection. In another embodiment, the pharmaceutical composition must be reconstituted prior to injection. For example, the pharmaceutical composition is reconstituted in water prior to injection.

In an embodiment, the pharmaceutical composition is administered in combination with an additional therapeutic agent. The administration of the additional therapeutic agent may be in the same composition or in separate compositions. The additional therapeutic agent may be a parasiticide or a vaccine.

In another embodiment, the additional therapeutic agent is another parasiticide. In an embodiment the parasiticide is macrocyclic lactone. In an embodiment macrocyclic lactone is moxidectin or milbemycin.

The other active ingredients are selected from the group consisting of isoxazoline compounds, avermectins (e.g., ivermectin, selamectin, doramectin, abamectin, and eprinomectin); milbemycins (moxidectin and milbemycin oxime); pro- benzimidazoles (e.g., febantel, netobimin, and thiophanate); benzimidazole derivatives, such as a thiazole benzimidazole derivatives (e.g., thiabendazole and cambendazole), carbamate benzimidazole derivatives (e.g., fenbendazole, albendazole (oxide), mebendazole, oxfendazole, parbendazole, oxibendazole, flubendazole, and triclabendazole); imidazothiazoles (e.g., levamisole and tetramisole); tetrahydropyrimidine (morantel and pyrantel), salicylanilides (e.g., closantel, oxyclozanide, rafoxanide, and niclosamide); nitrophenolic compounds (e.g., nitroxynil and nitroscanate); benzenedisulfonamides (e.g., clorsulon); pyrazinoisoquinolines (e.g., praziquantel and epsiprantel); heterocyclic compounds (e.g., piperazine, diethylcarbamazine, and phenothiazine); dichlorophen, arsenicals (e.g., thiacetarsamide, melorsamine, and arsenamide); cyclooctadepsipeptides (e.g., emodepside); paraherquamides (e.g. derquantel); and amino-acetonitrile compounds (e.g. monepantel, AAD 1566); amidine compounds (e.g., amidantel and tribendimidin), including all pharmaceutically acceptable forms, such as salts, solvates or N-oxides.

In an embodiment, the moxidectin is present in an amount between about 0.1%w/v to about 1.0%w/v.

An embodiment of the invention is a method of treating or preventing a parasite infestation in an animal comprising administering to an animal in need thereof an effective amount of the injectable pharmaceutical compositions described above.

In an embodiment, the animal suffers minimal injection site irritation. As noted above, minimal injection site irritation means injection site irritation that is less than 2 x 2 cm that persists for less than two to three days.

In an embodiment, the animal is a companion animal.

In an embodiment, the companion animal is a dog or cat.

The optimum amount to be employed for best results will, of course, depend upon the particular isoxazoline compound employed, the species of animal to be treated, the route and formulation of administration, and the type and severity of parasitic infection or infestation. Generally good results are obtained with isoxazoline compounds of formula (I) when administered from about 0.01 and 200 mg/kg body weight of the animal, in one embodiment 0.1 to 100 mg per kg of animal body weight, or 0.5 to 50 mg per kg of animal body weight or 1 to 30 mg per kg of animal body weight such total dose being given at one time or in divided doses.

It will be understood by the artisan that the methods of the present invention are useful in treating diseases and disorders that are known to be associated with the presence of arthropod parasites, including for example, those listed above. Such diseases (parasitoses) e.g., result from heavy parasite infestation, such as infestation of dogs with a high number of ticks in regions where the pressure from ticks is of such nature as to result in pathogenic consequences for the animal on a significant scale.

As used herein, the terms, "administer" or "administration" refer to the delivery of compound of Formula (I), a salt, solvate, or prodrug thereof, or of a pharmaceutical composition containing compound of Formula (I), a salt, solvate, or prodrug, to an animal for the purpose of controlling a parasite infestation in or on animals.

Administration of the inventive compounds may be intermittent and may be administered daily, weekly, biweekly, monthly, bimonthly, quarterly, half yealy, yearly or even at a lower frequency. The time period between treatments depends upon factors such as the parasite(s) being treated, the degree of infestation, the type of animal, mammal or bird, and the environment where it resides.

The injectable pharmaceutical compositions may be administered daily, weekly, monthly, semiannually or annually. The injectable pharmaceutical compositions may be administered every month, every two months, every three months, every four months, every five months, every 6 months, every seven months, eight months, every nine months, every ten months, every eleven months, every twelve months, every 13 months, every 14 months, every 15 months, every 16 months, every 17 months or every 18 months.

In an embodiment, the injectable isoxazoline pharmaceutical composition is administered with a separate injectable parasiticide composition. In an embodiment, the administration is simultaneous or sequential.

An embodiment of the injectable pharmaceutical composition, the D50 of particle size of the isoxazolne compound is from about 75 microns to about 130 microns and the D10 of the particle size is from about 30 microns to about 50 microns.

An embodiment of the invention is a kit treating or preventing a parasite infestation in an animal, the kit comprising
two or more containers
a) solid crystalline isoxazoline compound;
b) a vehicle comprising a pharmaceutically acceptable excipient capable of forming a suspension with the compound of a); and
c) instructions for combining the solid crystalline isoxazoline compound with the vehicle prior to injection.

wherein for the solid crystalline isoxazoline compound, the D50 of particle size is from about 75 microns to about 130 microns and the D10 of the particle size is from about 30 microns to about 50 microns.
IN another embodiment, the isoxazoline compound is fluralaner

Another embodiment is a method of treating or preventing a parasite infestation in an animal comprising administering to an animal in need thereof the above kit.

In additional embodiment, the injectable pharmaceutical composition is to be reconstituted with a vehicle.

In an additional embodiment, the vehicle comprises a diluent and optionally comprises a wetting agent, a antifoaming agent, a ph control agent, and/or a suspending agent.

### EXAMPLES

### Example 1 - fluralaner ready to use injectable suspension

**Table 3**

| Example | Composition % w/v (role) | API size (volume weighted particle size) |
|---|---|---|
| 1AA | 7.5% fluralaner, 0.25% NaCMC, | Micronized (around 5 µm |
| | 0.1%Lutrol, 2% benzyl alcohol, 0.2 Simethicone, 0.7% Sodium phosphate, HCl, qs H₂O | |
| 1A | 7.5% fluralaner (api), 0.25% NaCMC (suspending agent), 0.1%Lutrol L-44 (wetting agent), 2% benzyl alcohol (preservative), 0.2% Simethicone (anti-foaming agent), 0.7% Na Phosphate, HCl, H₂O | 10 µm (non-micronized) |
| 1B | 7.5% fluralaner(api), 0.25% NaCMC (suspending agent), 0.1%Lutrol, 2% benzyl alcohol, 0.7% Na Phosphate, HCl, H₂O | 10 µm (non-micronized) |
| 1C | 7.5% fluralaner(api), 0.25% NaCMC(suspending agent), 0.1%Lutrol, 2% benzyl alcohol, | 10 µm (non-micronized) |
| | 0.7% Na Phosphate, HCl, H₂O | |
| 1D | 7.5% fluralaner(api), 0.25% NaCMC(suspending agent), 0.1%Lutrol, 2% benzyl alcohol, | 40 µm(non-micronized) |
| | 0.7% Na Phosphate, HCl, H₂O | |
| 1E | 7.5% fluralaner(api), 0.5% NaCMC(suspending agent), 0.1% Lutrol, 2% benzyl alcohol, | 10 µm (non-micronized) |
| | 0.2% Simethicone (anti-foaming agent), 1.5% NaCitrate, HCl, H₂O | |
| 1F | 7.5% fluralaner(api), 0.5% NaCMC(suspending agent), 1% Lecithin (wetting agent), 2% benzyl alcohol, | 10 µm (non-micronized) |
| | 0.2% Simethicone(anti-foaming agent), 1.5% NaCitrate, HCl, H₂O | |
| 1G | 7.5% fluralaner(api), 0.5% NaCMC(suspending agent), 0.1% Lutrol, 2% BA, | 10 µm (non-micronized) |
| | 1.5% NaCitrate, HCl, H₂O | |
| 1H | 7.5% fluralaner(api), 0.5% NaCMC(suspending agent), 1% Lecithin, 2% BA, | 10 µm (non-micronized) |
| | 1.5% NaCitrate, HCl, H₂O | |
| 1I | 7.5% fluralaner(api), 0.5% NaCMC(suspending agent), 0.1% Lutrol, 2% benzyl alcohol, | 30 µm(non-micronized) |
| | 0.2% Simethicone(anti-foaming agent), 0.7% Na Phosphate, HCl, H₂O | |
| 1J | 7.5% fluralaner(api), 2.5% NaCMC(suspending agent), 0.1% Lutrol (surfactant/wetting agent), 1.5% benzyl alcohol, | 100 µm(non-micronized) |
| | 0.05% Simethicone(anti-foaming agent), H₂O | |
| Placebo | 7.5% Flur (micronized), 0.25% NaCMC, | No fluralaner |
| | 0.1%Lutrol, 2% benzyl alcohol, 0.2 Simethicone, 0.7% Sodium phosphate, HCl, qs H2O | |

The following procedure was used to produce formula of Example 1A
1. Charge -80% of the total volume of water for injection .
2. The suspending agent (Sodium carboxy methyl cellulose (NaCMC)) was added and mixed with an overhead mixer for ~ 5 minutes.
3. The mixture was further mixed with a homogenizer until free of agglomerates
4. The wetting agent (Poloxamer 124) was added and mixed with an overhead mixer until uniform .
5. The preservative (Benzyl alcohol (BA)) was added and mixed with an overhead mixer until uniform.
6. Sodium phosphate was added and mixed with an overhead mixer until uniform.
7. Fluralaner was added and mixed with a homogenizer until free of agglomerates.
8. The antifoaming agent (Simethicone) was mixed gently with an overhead mixer until uniform (5 minutes).
9. The pH of the mixture was adjusted to pH 7.0-7.4 with the addition of HCl. Mix gently with an overhead mixer until uniform (5 min).
10. Water was added QS to final weight for injection and then mixed gently with an overhead mixer until uniform (5 min).
11. The resulting formulation was packaged into injectable vials and sealed with stopper.
12. The vials were autoclaved for a cycle of 15 minutes at 121°C.

Analogous procedures were used to produce the formulas of Examples 1AA, 1B-1J and the placebo. Batch sizes ranged from 50 mL to 1000 mL. The volume weighted particle size of the fluralaner crystals (API) was measured by static light scattering (laser diffraction) (Sympatec Helos) to determine the particle size distribution.

### Example 2 - Injection site reaction evaluation

The administration sites were inspected prior to treatment on the day of treatment, 30 minutes following administration, one day after administration and then at intervals of 2-3 days until three weeks post administration. If a dog showed an administration site reaction at an evaluation time point, the study supervisor could decide on additional assessment time points. If a dog showed an administration site reaction at the last scheduled assessment time points, additional assessments were conducted on the individual dog at 2-3 day intervals until reactions have resolved.

The administration areas were first observed for swelling, erythema or other findings. Regardless of whether findings are observed, the administration areas were gently palpated for swelling, pain and increase in temperature. The following scoring system was used:
- Erythema, increase in temperature and pain:
   ∘ 0 = no reaction,
   ∘ 1 = slight reaction,
   ∘ 2 = moderate reaction,
   ∘ 3 = severe reaction.
- Swelling:
   ∘ size (measured with graduated equipment): length x width x height
   ∘ consistency: hard / soft
   ∘ freely movable: yes / no
   ∘ connection to surrounding tissue: discrete / diffuse

Other observations at the administration sites (e.g. crusts, wounds, scratches) were recorded in descriptive terms.

Each test group consisted of 6-10 dogs. The placebo group did not receive any fluralaner. The data below present the number of dogs with injection site irritation, a description of the size of the irritation in cm and the duration of the irritation.

**Table 4 Injection site irritation**

| Example | **Day 0-2** | **Day 3** - **5** | **Day 7** - **9** | **Day 12 - 14** | **Day 18** - **19** | **Day 20** - **21** |
|---|---|---|---|---|---|---|
| 1AA (5 µm) | swelling resolved, 2 dogs swelling (3.8x.8), 1 doq | | swelling returned, 1 dog swelling (2.8x3.6), 1 dog | swelling resolved, 1 dog swelling (3x4), 2 dog | | |
| 1A (10 µm) | 3x3.2, 1 dog 1.8x2, 1 dog | 3x3.2, 1 dog 1.8x2, 1 dog | resolved, 1 dog 2.5x3, 1 dog | 3x3.2, 1 dog 1.8x2, 1 dog | | |
| 1B (10 µm) | 2x2, 2 dogs | Resolved | | none | None | |
| 1C (10 µm) | 1.5x2, 7 dogs | | | | | 4 dogs unresolv ed |
| 1D (40 µm) | 2x1, 6 dogs | | | | | 4 dogs unresolv ed |
| 1E (10 µm) | 1x1, 2 dogs | 1x1, 2 dogs | | | | 1x1, 2 dogs |
| 1F (10 µm) | 2x4, 1 dog | | | 2x4,1 dog | 2x4,1 dog | 2x4,1 dog |
| 1G (10 µm) | | | 0.5x0.5, 1 dog | 0.5x0.5, 1 dog | | 0.5x0.5, 1 dog |
| 1H (10 µm) | 1x1, 2 dogs | | 1x1,2 dogs | | | 1x1, 2 dogs |
| 1I (10 µm) | 2x1, 1 dog | | | | | None |
| 1J (100 µm) | 1x1, 1 dog (30 min) | | | | | None |
| Placebo | swelling resolved, 1 doq | | swelling resolved, 1 doq | swelling resolved, 1 doq | | |

These data indicate that injection site irritation of the composition with smaller fluralaner particle size was more severe that those with a larger particle size. Specifically, Formulation 1AA whose fluralaner was micronized to a particle size of about 5 µm produced injection site irritation of large swelling which persisted for 12-14 days while the larger particle Formulation 1J (particle size 100 µm) produced negliable swelling that lasted only a short duration. This was similar to the reaction of the placebo group.

### Example 3 - Pharmacokinetic Testing

Study A: Fluralaner 5 micron 7.5% suspension (Example 1AA) and Fluralaner 40 micron 7.5% suspension (Example 1D) were administered subcutaneously on a single occasion at 15 mg/kg body weight (BW) to eight Beagle dogs each. The local tolerance of the test articles was assessed at intervals up to 28 days after administration. Blood samples for determination of fluralaner plasma concentrations were collected prior to treatment, at 2 hours and 8 hours, and at 1, 2, 3, 5, 7, 10, 14, 21, 28, 35, 42, 49, 56, 70, 84, 98, 112, 126, 140 and 154 days post treatment.

Study B: Fluralaner 100 micron 7.5% suspension (Example 1J) was administered subcutaneously on a single occasion at 10 mg/kg BW to three Beagle dogs. The local tolerance of the test articles was assessed at intervals up to 28 days after administration. Blood samples for determination of fluralaner plasma concentrations were collected prior to treatment, and at 1, 3, 5, 7, 10, 14, 21, 28, 35, 49, 56, 63, and 70 days post treatment.

The initial absorption of all formulations is comparable during the first days suggesting similar onset of efficacy. The 100 micron suspension at 10 mg/kg BW shows a lower plasma profile which is more favorable in terms of target animal safety. At the same time, plasma concentrations are high enough to suggest sufficient tick efficacy. The 40 micron suspension, compared to the micronized suspension administered at the same dose, shows a higher plasma concentration at time points after day 84, and is expected to provide longer duration of tick efficacy.

Overall, a large particle provides blood levels of fluralaner that are sufficient to indicate a long duration of tick efficacy while showing a lower risk for adverse events such as injection site irritation.

### Example 4 - Efficacy Testing

### Example 4 A

Dose characterization studies were conducted to define the effective dose using *Rhipicephalus sanguineus* and *Amblyomma americanum* on dogs. Doses of 10, 15 and 20 mg fluralaner/kg body weight were evaluated. These studies consisted of an untreated control group and three fluralaner treated groups, each group containing 8 to10 dogs. Individual dogs were infested with 50 ticks of each species prior to treatment and then on specified post treatment days. Effectiveness was determined by counting the live ticks on all dogs approximately 48 to 72 hours following infestation and calculating the % efficacy or % reduction in live ticks relative to the untreated control group. The results from one of the ongoing studies is presented in Table 5 and 6.

**Table 5. Effectiveness of fluralaner injectable suspension administered at 10, 15 or 20 mg fluralaner/kg body weight against R. sanguineus ticks on dogs^{a,b}**

| Study Day | Tick counts by fluralaner dose (mg/kg) | | | | % efficacy by fluralaner dose (mg/kg) | | |
|---|---|---|---|---|---|---|---|
| | 0 | 10 | 15 | 20 | 10 | 15 | 20 |
| 2 | 32.3 | 26.4 | 20.3 | 12.3 | 18.2 | 37.2 | 62.0 |
| 14 | 21.5 | 0.3 | 0.0 | 0.0 | 98.8 | 100 | 100 |
| 32 | 28.8 | 0.0 | 0.0 | 0.0 | 100 | 100 | 100 |
| 62 | 24.1 | 0.0 | 0.0 | 0.0 | 100 | 100 | 100 |
| 92 | 30.6 | 0.0 | 0.0 | 0.0 | 100 | 100 | 100 |
| 123 | 30.5 | 0.1 | 0.1 | 0.0 | 99.5 | 99.6 | 100 |
| 153 | 29.4 | 0.9 | 0.0 | 0.0 | 97.1 | 100 | 100 |
| 183 | 31.4 | 0.6 | 0.0 | 0.3 | 98.2 | 100 | 99.1 |
| 212 | 31.1 | 0.3 | 0.1 | 0.1 | 99.1 | 99.6 | 99.6 |
| 242 | 32.6 | 1.9 | 0.5 | 0.0 | 94.3 | 98.5 | 100 |
| 274 | 34.9 | 0.6 | 0.3 | 0.1 | 98.4 | 99.3 | 99.6 |
| 302 | 30.7 | 2.6 | 0.6 | 0.0 | 91.6 | 98.0 | 100 |
| 333 | 32.1 | 4.1 | 0.1 | 0.3 | 87.1 | 99.6 | 99.2 |
| 363 | 33.7 | | 5.5 | 1.3 | | 83.7 | 96.3 |
| 392 | 27.6 | | 12.4 | 12.0 | | 55.1 | 56.5 |

| | | | | | | | |
|---|---|---|---|---|---|---|---|
| ^{a}Counts are arithmetic means of live ticks and % efficacy was calculated with arithmetic means. ^{b}Counts were done 48 h following treatment or infestation. | | | | | | | |

**Table 6. Effectiveness of fluralaner injectable suspension administered at 10, 15 or 20 mg fluralaner/kg body weight against A. americanum ticks on dogs ^{a,b}**

| Study Day | Tick counts by fluralaner dose (mg/kg) | | | | % efficacy by fluralaner dose (mg/kg) | | |
|---|---|---|---|---|---|---|---|
| | 0 | 10 | 15 | 20 | 10 | 15 | 20 |
| 2 | 25.8 | 22.8 | 20.3 | 15.6 | 20.2 | 28.9 | 45.2 |
| 14 | 20.4 | 0.4 | 0.4 | 1.0 | 98.2 | 98.2 | 95.1 |
| 32 | 20.3 | 0.0 | 0.0 | 0.0 | 100 | 100 | 100 |
| 62 | 19.8 | 0.1 | 0.0 | 0.0 | 99.3 | 100 | 100 |
| 92 | 17.9 | 0.1 | 0.0 | 0.0 | 99.2 | 100 | 100 |
| 123 | 13.6 | 0.9 | 0.0 | 0.4 | 93.7 | 100 | 96.9 |
| 161 | 31.8 | 4.3 | 2.0 | 2.3 | 86.5 | 93.7 | 92.8 |
| 183 | 25.5 | 6.6 | 1.6 | 0.2 | 74.2 | 93.6 | 99.3 |
| 212 | 26.6 | 8.4 | 5.9 | 7.4 | 68.3 | 77.9 | 72.3 |

| | | | | | | | |
|---|---|---|---|---|---|---|---|
| ^{a}Counts are arithmetic means of live ticks and % efficacy was calculated with arithmetic means. ^{b}Counts up to and including Day 161 were done at 48 h following treatment or infestation. Counts on Days 183 and 212 were at 72 h. | | | | | | | |

In the examples, *Amblyomma americanum* and *Rhipicephalus sanguineus* efficacy was tested because it is known that these parasite species are the dose limiting ticks (i.e., an effective dose against these ticks will also be effective against other tick species) and that from this date it can be expected that efficacy against fleas ( more than 90% mortality) can be achieved by administration of a composition involving isoxazoline compounds of formula (I) at least as long as activity against *Amblyomma americanum* and *Rhipicephalus sanguineus* was observed .

Therefore the compositions according to the current invention are effective to control flea infestations of companion animals, especially dogs and cats at least as long as *Amblyomma americanum* and *Rhipicephalus sanguineus,* i.e. at least 90 days.

### Example 4 B

The results from additional dose characterization studies are summarized. These studies evaluated three doses (10, 15 and 20 mg/kg) in adult dogs. The injectable suspension formulations of fluralaner were prepared is indicated in Example 1J above. One study tested the effectiveness against *Rhipicephalus sanguineus* ticks (see Table 7). A second study evaluated efficacy against fleas at approximately 6 months after treatment was tested in dogs (see Table 8).

**Table 7. Effectiveness of fluralaner injectable suspension administered at 10, 15 or 20 mg fluralaner/kg body weight against R. sanguineus ticks on dogs ^{a,b}**

| Study Day | Tick counts by fluralaner dose (mg/kg) | | | | % efficacy by fluralaner dose (mg/kg) | | |
|---|---|---|---|---|---|---|---|
| | 0 | 10 | 15 | 20 | 10 | 15 | 20 |
| 2 | 40.8 | 27.6 | 15.3 | 20.8 | 32.2 | 62.6 | 49.1 |
| 9 | 38.5 | 6.0 | 3.0 | 1.8 | 84.4 | 92.2 | 95.5 |
| 16 | 37.3 | 1.5 | 2.3 | 1.1 | 96.0 | 94.0 | 97.0 |
| 30 | 35.5 | 2.5 | 1.9 | 1.8 | 93.0 | 94.7 | 95.1 |
| 58 | 31.1 | 1.5 | 1.1 | 1.4 | 95.2 | 96.4 | 95.6 |
| 86 | 38.6 | 1.1 | 1.9 | 1.4 | 97.1 | 95.1 | 96.4 |
| 114 | 47.3 | 0.1 | 0.5 | 0.4 | 99.7 | 98.9 | 99.2 |
| 142 | 41.6 | 0.1 | 0.1 | 0.3 | 99.7 | 99.7 | 99.4 |
| 170 | 38.8 | 0.1 | 0.4 | 1.5 | 99.7 | 99.0 | 96.1 |
| 184 | 40.9 | 0.3 | 0.8 | 0.8 | 99.4 | 98.2 | 95.1 |
| 212 | 38.6 | 2.0 | 1.9 | 0.6 | 94.8 | 98.2 | 98.4 |

| | | | | | | | |
|---|---|---|---|---|---|---|---|
| ^{a}Counts are arithmetic means of live ticks and % efficacy was calculated with arithmetic means. ^{b}Counts were done 48 h following treatment or infestation. | | | | | | | |

**Table 8. Effectiveness of fluralaner injectable suspension administered at 10, 15 or 20 mg fluralaner/kg body weight against Ctenocephalides felis fleas on dogs ^{a,b}**

| Study Day | Flea counts by fluralaner dose (mg/kg) | | | | % efficacy by fluralaner dose (mg/kg) | | |
|---|---|---|---|---|---|---|---|
| | 0 | 10 | 15 | 20 | 10 | 15 | 20 |
| 187 | 89.4 | 0.0 | 0.0 | 0.0 | 100 | 100 | 100 |

| | | | | | | | |
|---|---|---|---|---|---|---|---|
| ^{a}Counts are arithmetic means of live fleas and % efficacy was calculated with arithmetic means. ^{b}Counts were done at 24 h following infestation. | | | | | | | |

Results from these studies demonstrated effectiveness of fluralaner in an injectable suspension against *R. sanguineus* for at least 7 and 12 months, respectively.

Efficacy of the 15 and 20 mg/kg doses were also demonstrated for 6 months following injection against *A. americanum.*

All three doses were demonstrated to be 100% effective in reducing live fleas on dogs at 6 months following treatment. Efficacy against fleas would be predicted to last 12 months as it has been demonstrated that fleas are more sensitive to fluralaner than ticks. This is especially true for the dose limiting species, *R. sanguineus* and A. *americanum.*

### Example 5 - fluralaner reconstitutable injectable formulations

Additional examples of injectable fluralaner suspension formulation were prepared as reconstitutable formulations. The following procedure was used to produce Formula 5A

### A. The vehicle

1. Charge -80% of the total volume of water for injection.
2. The suspending agent (Sodium carboxy methyl cellulose (NaCMC)) was added and mixed with an overhead mixer for ~ 5 minutes.
3. The mixture was further mixed with a homogenizer until free of agglomerates
4. The wetting agent (Poloxamer 124) was added and mixed with an overhead mixer until uniform .
5. The preservative (Benzyl alcohol (BA)) was added and mixed with an overhead mixer until uniform.
6. Sodium phosphate was added and mixed with an overhead mixer until uniform.
7. The antifoaming agent (Simethicone) was mixed gently with an overhead mixer until uniform (5 minutes).
8. The pH of the mixture was adjusted to pH 7.0-7.4 with the addition of HCl. Mix gently with an overhead mixer until uniform (5 min).
9. Water was added QS to final weight for injection and then mixed gently with an overhead mixer until uniform (5 min).
10. The resulting formulation was packaged into injectable vials and sealed with stopper.
11. The vials were autoclaved for a cycle of 15 minutes at 121°C.

### B. The active ingredient

1. solid fluralaner was added to a vial and sealed.
2. The vial was terminally sterilized by gamma radiation.

### C. Formation of the reconstituted injectable formulation

1. The vehicle of vial of A is added to the active ingredient vial of B and shaken
2. The resultant suspension was ready for injection.

Analogous procedures were used to produce the formulations of Examples 5B-H. Batch sizes ranged from 50 mL to 1000 mL.

The volume weighted particle size of the fluralaner crystals (API) was measured by static light scattering (laser diffraction) (Sympatec Helos) to determine the particle size distribution.

The composition of Formulations 5A-H are given on Table 9

**Table 9**

| Example | Material | % w/v | API size in µm (volume weighted particle size) D10/D50 |
|---|---|---|---|
| 5A | LPS Fluralaner | 15.00 | 20/100 |
| | Sodium CMC (Blanose 7M8 SF PH) | 2.00 | |
| | Poloxamer 124 | 0.10 | |
| | Benzyl alcohol | 2.00 | |
| | Simethicone emulsion | 0.20 | |
| | Sodium phosphate (dibasic dihydrate) | 0.70 | |
| | Hydrochloric acid (1N) | as needed for pH adjustment | |
| | Water for injection | QS | |
| 5B | Same as 5A except api size | | 40/100 |
| 5C | Same as 5A except api size | | 50/130 |
| 5D | Irradiated, same as 5B except no poloxamer, | | 40/100 |
| 5E | Same as 5B except 2.3 % NaCMC | | 40/100 µm |
| 5F | Same as 5B except 0.05% poloxamer | | 40/100 µm |
| 5G | Same as 5A except api size | | D50 is 77 µm |
| 5H | Same as 5A except api size | | D50 is 116 µm |

### Example 6 - Pharmacokinetic Testing of fluralaner reconstitutable injectable formulations

The evaluation of the pharmacokinetic properties of the formulations of Example 5 was conducted as described in Example 3 except as noted below.

### Example 6A:

Samples of Formulations 5B (fluralaner, D50 = 100 micron, 15% suspension, 5B (irradiated), and 5D (irradiated, no poloxamer) were administered subcutaneously on a single occasion at 20 mg/kg BW to eight, sixteen and sixteen Beagle dogs, respectively. The local tolerance of the test articles was assessed at intervals up to 21 days after administration. Blood samples for determination of fluralaner plasma concentrations were collected prior to treatment, at 8 hours, and at 1, 3, 5, 7, 10, 14, 21, 28, 35, 42, 49, 56, 70, 84, 98, 112, 126, 140, 154, 168, and 182 days post treatment.

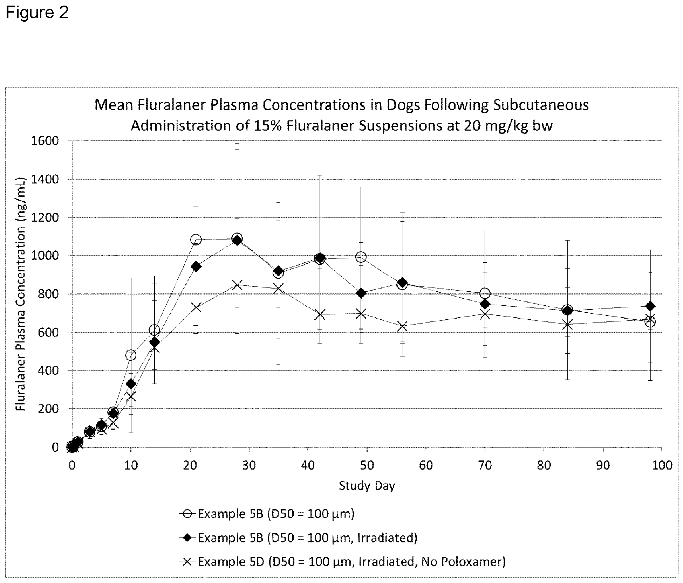

See Figure 2.

### Example 6B:

Samples of Formulations 5A (Fluralaner 100 micron (D10=20, D50=100) 15% suspension), 5B fluralaner 100 micron (D10=40, D50=100) 15% suspension, and 5C (fluralaner 130 micron (D10=50, D50=130) 15% suspension) were administered subcutaneously on a single occasion at 20 mg/kg BW to 10 Beagle dogs each. The local tolerance of the test articles was assessed at intervals up to 21 days after administration. Blood samples for determination of fluralaner plasma concentrations were collected prior to treatment, at 8 hours, and at 1, 3, 5, 7, 10, 14, 21, 28, 35, 42, 49, 56, 70, 84, 98, 112, 126, 140, 154, 168 and 182 days post treatment.

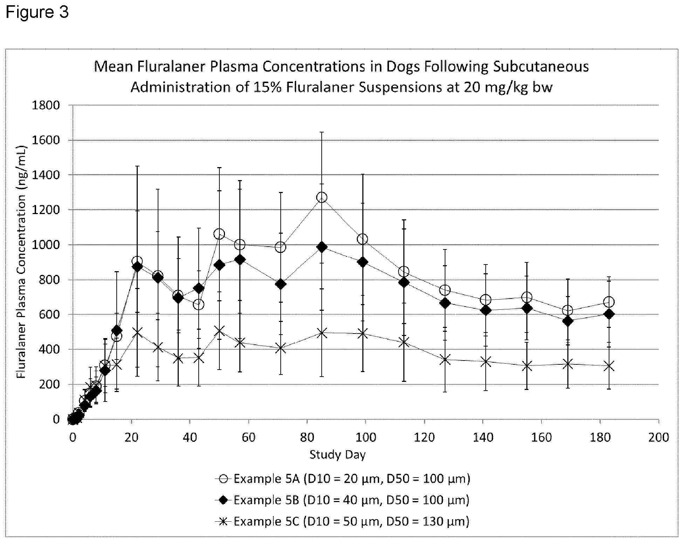

See Figure 3

### Example 6C:

Samples of Formulations 5B (Fluralaner 100 micron 15% suspension), 5E ( fluralaner 100 micron 15% suspension with 2.3% NaCMC), 5F (fluralaner 100 micron 15% suspension with 0.05% poloxamer), 5G (fluralaner 77 micron 15% suspension), and 5H (fluralaner 116 micron suspension) were administered subcutaneously on a single occasion at 20 mg/kg BW to 10 Beagle dogs each. The local tolerance of the test articles was assessed at intervals up to 21 days after administration. Blood samples for determination of fluralaner plasma concentrations were collected prior to treatment, at 8 hours, and at 1, 3, 5, 7, 10, 14, 21, 28, 35, 42, 49, 56, 70, 84, 98, 112, 126, 140, 154, 168 and 182 days post treatment.

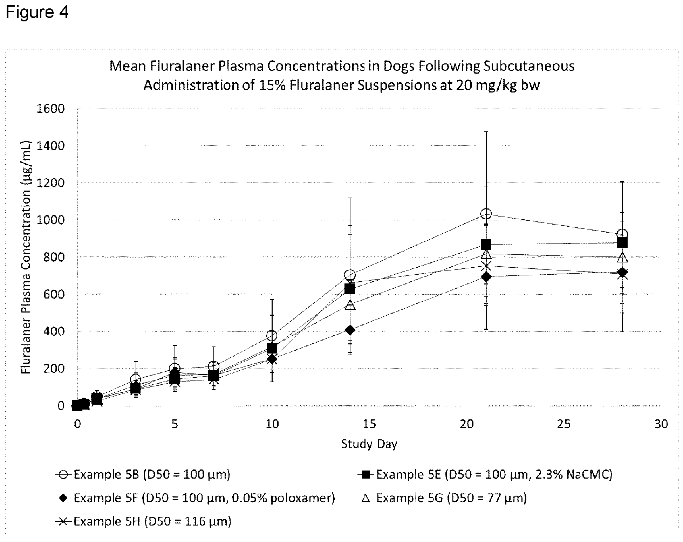

See Figure 4

### Example 7 - Injection site reaction evaluation of reconstitutable formulations

Evaluation of the formulations of Example 5 was conducted as described in Example 2.

These formulations were evaluated during the pharmacokinetic experiments described in Example 6. There were no significant injection reactions during the evaluation of the formulations of Example 5.

## Claims

1. An injectable pharmaceutical composition comprising particles of an isoxazoline compound of Formula (I) 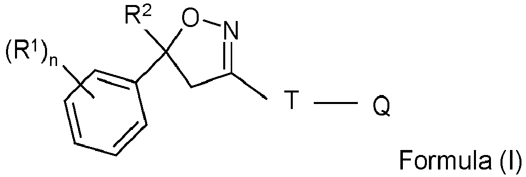 wherein
R¹ is halogen, CF₃, OCF₃, or CN;
n is an integer from 0 up to and including 3, preferably 1, 2 or 3:
m is 1 or 2;
R² is C₁-C₃ haloalkyl, CF₃ or CF₂Cl
T is a 5, or 6 membered ring, or bicyclic, which is optionally substituted by one or more radicals Y;
Y is methyl, halomethyl, halogen, CN, NO₂, NH₂-C=S, or two adjacent radicals Y together form a chain;
Q is X-NR³R⁴, NR⁵-NR⁶-X-R³, X-R³, or a 5-membered N-heteroaryl ring, which is optionally substituted by one or more radicals;
X is CH₂, CH(CH₃), CH(CN), CO, CS;
R³ is hydrogen, methyl, haloethyl, halopropyl, halobutyl, methoxymethyl, methoxyethyl, halomethoxymethyl, ethoxymethyl, haloethoxymethyl, propoxymethyl, ethylaminocarbonylmethyl, ethylaminocarbonylethyl, dimethoxyethyl, propynylaminocarbonylmethyl, N-phenyl-N-methyl-amino,
haloethylaminocarbonylmethyl, haloethylaminocarbonylethyl, tetrahydrofuryl, methylaminocarbonylmethyl, (N,N-dimethylamino)-carbonylmethyl, propylaminocarbonylmethyl, cyclopropylaminocarbonylmethyl, propenylaminocarbonylmethyl, haloethylaminocarbonylcyclopropyl, alkylsulfanylalkyl, alkylsulfinylalkyl, alkylsulfonylalkyl, cycloalkyl, 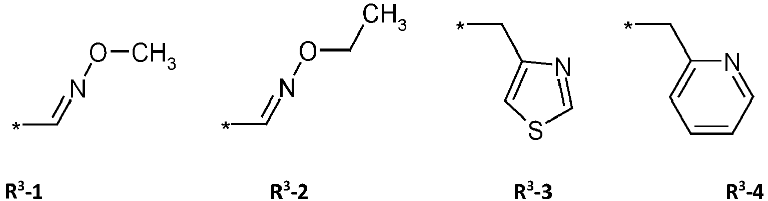 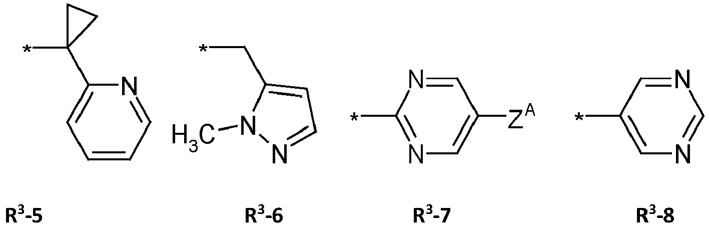 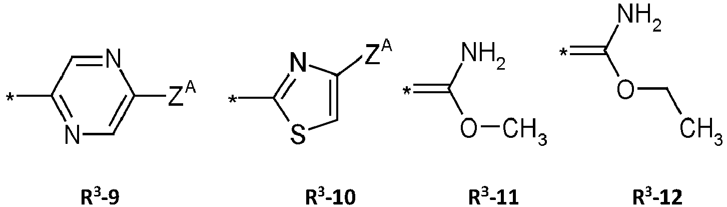 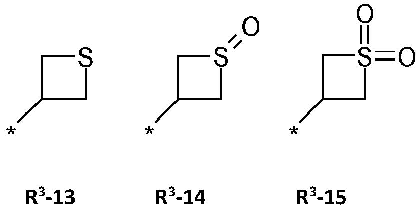 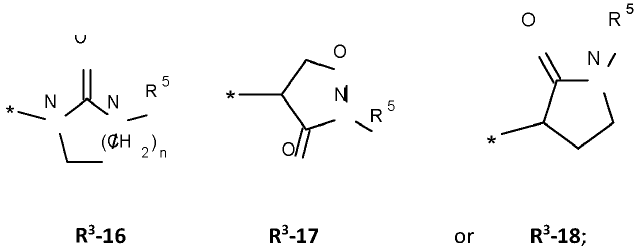
wherein
Z^{A} is hydrogen, halogen, cyano, or halomethyl (CF₃);
R⁴ is hydrogen, ethyl, methoxymethyl, halomethoxymethyl, ethoxymethyl, haloethoxymethyl, propoxymethyl, methylcarbonyl, ethylcarbonyl, propylcarbonyl, cyclopropylcarbonyl, methoxycarbonyl, methoxymethylcarbonyl, aminocarbonyl, ethylaminocarbonylmethyl, ethylaminocarbonylethyl, dimethoxyethyl, propynylaminocarbonylmethyl, haloethylaminocarbonylmethyl, cyanomethylaminocarbonylmethyl, or haloethylaminocarbonylethyl;
R⁵ is H, alkyl, or haloalkyl;
R⁶ is H, alkyl, or haloalkyl;
or wherein R³ and R⁴ together form a substituent selected from the group consisting of: 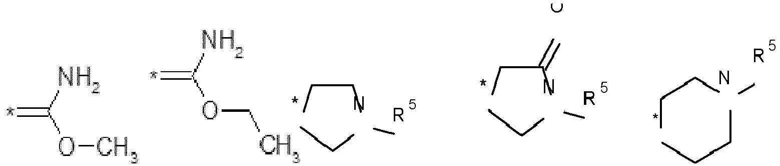 and 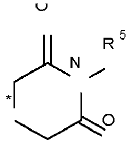
or a salt or N-oxide thereof and a pharmaceutical acceptable excipient wherein the isoxazoline compound has a volume weighted particle size distribution D50 as measured by a static light scattering instrument of from about 50 microns to about 150 microns.

2. The injectable pharmaceutical composition of claim 1, wherein the isoxazoline compound is in suspension in the composition.

3. The injectable pharmaceutical composition of any of claims 1-2, wherein the particle size distribution D50 is from about 75 microns to about 150 microns.

4. The injectable pharmaceutical composition of any of claims 1-3, wherein the particle size distribution D50 is from about 90 microns to about 110 microns.

5. The injectable pharmaceutical composition of any of claims 1-4, wherein the D50 of volume weighted particle size distribution is from about 75 microns to about 130 microns and the D10 of the particle size is from about 30 microns to about 50 microns.

6. The injectable pharmaceutical composition of any of claims 1-5, wherein the isoxazoline compound is fluralaner.

7. The injectable pharmaceutical composition of any of claims 1-6, wherein the isoxazoline compound is present in an amount between about 5% w/v to about 50% w/v.

8. The injectable pharmaceutical composition of any of claims 1-7, wherein the composition is to be reconstituted with a vehicle.

9. The injectable pharmaceutical composition of any of claims 1-8, wherein the composition further comprises another parasiticide compound.

10. The injectable pharmaceutical composition of claim 9, wherein the parasiticide compound is a macrocyclic lactone.

11. The injectable pharmaceutical composition of claim 9, wherein the parasiticide compound is moxidectin.

12. The injectable pharmaceutical composition of claim 11, wherein the moxidectin is present in an amount between about 0.1%w/v to about 1.0%w/v.

13. The injectable pharmaceutical composition of any of claims 1-12 for use in the treatment or prevention of a parasite infestation in an animal .

14. The use of claim 13, wherein the animal is a dog.

15. The use of any one of claims 13-14, wherein the injectable isoxazoline pharmaceutical composition is administered simultaneous or sequential with a separate injectable parasiticide composition.

16. A kit for treating or preventing a parasite infestation in an animal, the kit comprising two or more containers
a) solid crystalline isoxazoline compound;
b) a vehicle comprising a pharmaceutically acceptable excipient capable of forming a suspension with the compound of a); and
c) instructions for combining the solid crystalline isoxazoline compound with the vehicle prior to injection.
wherein for the solid crystalline isoxazoline compound, the D50 of volume weighted particle size is from about 75 microns to about 130 microns and the D10 of the particle size is from about 30 microns to about 50 microns.

17. The kit of claim 16, wherein the isoxazoline compound is fluralaner.
